(19) Europäisches Patentamt
European Patent Office
Office européen des brevets

(11) EP 3 509 652 B1

(12) **FASCICULE DE BREVET EUROPEEN**

(45) Date de publication et mention de la délivrance du brevet:
**13.03.2024 Bulletin 2024/11**

(21) Numéro de dépôt: **17771502.6**

(22) Date de dépôt: **08.09.2017**

(51) Classification Internationale des Brevets (IPC):
***A61L 27/36*** (2006.01) ***A61L 27/44*** (2006.01)
***A61L 27/58*** (2006.01)

(52) Classification Coopérative des Brevets (CPC):
**A61L 27/44; A61L 27/3604; A61L 27/58**

(86) Numéro de dépôt international:
**PCT/FR2017/052395**

(87) Numéro de publication internationale:
**WO 2018/046870 (15.03.2018 Gazette 2018/11)**

(54) **BIOMATERIAU A USAGE THERAPEUTIQUE**

BIOMATERIAL ZUR THERAPEUTISCHEN VERWENDUNG

BIOMATERIAL FOR THERAPEUTIC USE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **09.09.2016 FR 1658416**

(43) Date de publication de la demande:
**17.07.2019 Bulletin 2019/29**

(73) Titulaires:
- **Assistance Publique-Hôpitaux de Paris (AP-HP) 75004 Paris (FR)**
- **Institut National de la Santé et de la Recherche Médicale (INSERM) 75013 Paris (FR)**
- **Université Paris Cité 75006 Paris (FR)**

(72) Inventeurs:
- **MENASCHE, Philippe 75006 Paris (FR)**
- **RENAULT, Nisa Middleton, Wisconsin 53562 (US)**
- **BELLAMY, Valérie 94240 L'Hay-les-Roses (FR)**
- **PIDIAL, Laetitia 77350 Le Mée-sur-Seine (FR)**

(74) Mandataire: **Novagraaf Technologies**
**Bâtiment O2**
**2, rue Sarah Bernhardt**
**CS90017**
**92665 Asnières-sur-Seine Cedex (FR)**

(56) Documents cités:
**WO-A1-2013/142878 WO-A1-2013/169202**

- **MENASCHÉ PHILIPPE: "The future of stem cells: Should we keep the "stem" and skip the "cells"?", THE JOURNAL OF THORACIC AND CARDIOVASCULAR SURGERY, MOSBY-YEAR BOOK, INC., ST. LOUIS, MO, US, vol. 152, no. 2, 2 March 2016 (2016-03-02) , pages 345-349, XP029640162, ISSN: 0022-5223, DOI: 10.1016/J.JTCVS.2016.02.058**
- **Fleury Audrey ET AL: "Extracellular Vesicles as Therapeutic Tools in Cardiovascular Diseases", Frontiers in Immunology, vol. 5, 4 August 2014 (2014-08-04), XP055791330, DOI: 10.3389/fimmu.2014.00370**
- **Olivier G. De Jong ET AL: "Extracellular Vesicles: Potential Roles in Regenerative Medicine", Frontiers in Immunology, vol. 5, 1 December 2014 (2014-12-01), XP055626541, DOI: 10.3389/fimmu.2014.00608**
- **Mahdi Salih: "Third International Meeting of ISEV 2014", ISEV meeting 2014, Rotterdam, Netherlands, 30 April 2014 (2014-04-30), XP055225255,**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



(Cont. page suivante)

- **P. A Janmey ET AL: "Fibrin gels and their clinical and bioengineering applications", The Lancet, vol. 236, no. 4, 6 January 2009 (2009-01-06), pages 126-10, XP055325053, AMSTERDAM, NL ISSN: 0140-6736, DOI: 10.1098/rsif.2008.0327**

## Description

### Domaine technique

La présente invention se rapporte à un biomatériau, à un procédé de fabrication du biomatériau.

La présente invention trouve par exemple des applications dans le domaine thérapeutique, chez l'homme et chez l'animal, notamment pour le traitement de tissus déficients.

Les références entre crochets « **[ ]** » ci-dessous renvoient à la liste de référence à la fin des exemples.

### Art antérieur

La thérapie cellulaire s'est rapidement avérée être un moyen efficace et à fort potentiel pour restaurer des tissus déficients du fait d'une maladie, d'un accident, d'une mutation génétique, de fonctions cellulaires défaillantes ou inopérantes, etc. Elle consiste à greffer des cellules dites « thérapeutiques » afin de restaurer la fonction d'un tissu ou d'un organe chez un patient. Les cellules thérapeutiques utilisées sont en fait des cellules obtenues à partir de cellules souches en général pluripotentes ou multipotentes qui peuvent provenir du patient lui-même ou d'un donneur. L'objectif de la thérapie cellulaire est de traiter le patient de préférence grâce à une injection de ces « cellules thérapeutiques » afin d'obtenir des résultats de restauration des tissus stables dans le temps.

La publication du Pr. Philippe Menasché et al., « Towards a clinical use of human embryonic stem cell-derived cardiac progenitors : a translational expérience », European Heart Journal (2015) 36, 743-750 **[1]** décrit par exemple un ensemble de recherches et de résultats obtenus par une technique de thérapie cellulaire visant le coeur sur des modèles de rats et de primates non-humains présentant un infarctus du myocarde. Les cellules utilisées sont des cellules progénitrices cardiaques issues de cellules souches embryonnaires humaines.

La recherche sur les cellules souches est toujours controversée, en particulier pour des questions éthiques, même si elle propose de nouvelles thérapies très utiles pour l'homme. Des recherches complémentaires sont encore nécessaires pour améliorer les techniques actuelles de thérapie cellulaire, notamment afin de trouver ou de perfectionner notamment les moyens actuels de génération, de culture et/ou de transformation des cellules utilisées, et de trouver des moyens permettant une injection ou un greffage chez le patient, malgré l'environnement cellulaire et les mécanismes naturels de rejet ou de dégradation. Il est aussi nécessaire de respecter le cadre juridique généralement contraignant imposé par les Etats pour ce type de recherche. En outre, les coûts impliqués par ce type de thérapie sont encore très importants.

On considère actuellement qu'un des mécanismes d'action présumé des cellules souches greffées serait une activation paracrine de voies de signalisation endogènes. Plusieurs études suggèrent que cette interaction paracrine entre cellules greffées et tissu hôte ne se limiterait pas à la seule sécrétion de facteurs solubles dans le milieu extracellulaire, mais impliquerait des vésicules issues des cellules. Des travaux récents ont été publiés à ce sujet, notamment dans Anaïs Kervadec, Pr. Philippe Menasché et al. « Cardiovascular progenitor-derived extracellular vesicles recapitulate the bénéficiai effect of their parent cells in the treatment of chronic heart failure », Elsevier, Original Pre-clinic Science, The Journal of Heart and Lung Transplantation, January 13, 2016; 35:795-807 **[2].** Ce document mentionne que des vésicules extracellulaires, incluant des exosomes et des microparticules sécrétées par des cellules transplantées, pourraient avoir un effet thérapeutique paracrine.

Le document WO2013/169202 (Biomatcell AB) publié le 14/11/2014 décrit l'utilisation de vésicules extracellulaires pour induire ou stimuler la différenciation de cellules souches en cellules osseuses, et la potentielle utilisation des vésicules dans un matériau biodégradable en vue d'améliorer la régénération osseuse ou l'ostéointégration d'implants.

Le document WO2013/142878 (NEOSTEM, Inc.) publié le 26/09/2013 a pour objet un procédé d'identification et de purification de population de cellules souches VSEL (Very Small Embryonic-Like stem cells), de vésicules produites par ces cellules et possédant des marqueurs à leur surface. Ce document décrit que des cellules VSEL ou des vésicules extracellulaires collectées à partir du sang d'un sujet par le procédé décrit peuvent être injectées chez un individu qui en a besoin, notamment pour le traitement de tissus cardiaques.

### Exposé de l'invention

La présente invention a notamment pour but de résoudre les inconvénients précités de l'art antérieur en fournissant une alternative à l'utilisation de cellules souches pour le traitement et/ou la restauration de tissus déficients, en particulier de tissus cardiaques, chez l'homme et chez l'animal, tout en bénéficiant des avantages fournis par la thérapie cellulaire, sans les inconvénients précités.

Les inventeurs de la présente sont en effet les tous premiers à avoir défini et mis en oeuvre que des combinaisons de polymère(s) biocompatible(s) et de vésicules extracellulaires de cellules souches permettent de restaurer des tissus déficients, en particulier de tissus cardiaques, ceci en l'absence de cellules souches.

Ainsi, la présente invention se rapporte en particulier à un biomatériau comprenant un polymère biocompatible biodégradable incluant des vésicules extracellulaires de cellules souches. La présente invention a pour objet un biomatériau comprenant un polymère biocompatible biodégradable incluant des vésicules extracellulaires de cellules souches pluripotentes ou multipotentes ou de cellules progénitrices cardiaques, lesdites vésicules étant un ou plusieurs éléments produits par lesdites cellules choisis parmi les microparticules, les exosomes et les corps apoptotiques, dans lequel ledit polymère biocompatible biodégradable est un polymère de fibrine ayant une taille des pores comprise entre 50 μm et 200 μm, une épaisseur comprise entre 0,8 mm et 1,2 mm et une élasticité comprise entre 6 kPa et 15 kPa.

Les inventeurs de la présente ont en effet notamment déterminé, dans le cadre de la présente invention, en particulier dans la combinaison précitée, que le biomatériau est capable de disparaître progressivement une fois implanté dans l'organisme, afin d'assurer une libération à la fois stable et prolongée dans le temps des vésicules incluses dans celui-ci, qui ne nuit pas à l'activité des vésicules incluses dans celui-ci, et qui permet un traitement prolongé des tissus déficients à la fois économique, exempt de questions éthiques, qui s'avère efficace et qui ne nécessite pas de nouvelle intervention spéciale de retrait ou remplacement du matériau implanté.

Selon l'invention, par « polymère », on entend un polymère organique ou inorganique ou une matrice organique, notamment cellulaire décellularisée, ou inorganique ou un mélange polymère organique ou inorganique et matrice organique ou inorganique, le polymère pouvant lui-même être considéré comme une matrice dans le cadre de la présente invention puisqu'il est avantageusement capable d'inclure et de libérer progressivement des vésicules de cellules souches, en particulier pour le traitement d'un tissu déficient chez un patient, humain ou animal. Des exemples de polymères sont présentés ci-dessous.

Par polymère « biocompatible », on entend un polymère qui est avantageusement à la fois compatible pour une implantation chez un patient, c'est-à-dire que cette implantation présente un rapport bénéfice / risque favorable du point de vue thérapeutique, par exemple au sens de la Directive 2001/83/CE, c'est-à-dire un risque réduit voire inexistant pour le patient, versus le bénéfice thérapeutique concerné ; et compatible pour y inclure des vésicules de cellules souches, c'est-à-dire qui permet l'inclusion de vésicules, qui ne dégrade pas ou peu l'activité des vésicules incluses dans le polymère et/ou la matrice biocompatible, et qui libère lesdites vésicules une fois que le biomatériau est implanté chez un patient, humain ou animal.

Par polymère « biodégradable », on entend biorésorbable et/ou biodégradable et/ou bioabsorbable, avec un but commun de disparition progressive et, ce faisant, de libération progressive des vésicules incluses dans le polymère biocompatible, avec un ou des mécanismes différents ou complémentaires de dissolution ou d'absorption du polymère chez le patient, humain ou animal, chez lequel le matériau a été implanté. Ainsi, suivant le choix du polymère parmi ceux déterminés par les inventeurs comme convenant à la mise en oeuvre de la présente invention, la dissolution peut être liée au matériau lui-même qui se dissout progressivement et/ou liée à des mécanismes d'élimination du corps humain ou animal, notamment par le biais des enzymes présents dans les fluides corporels.

Un polymère biocompatible biodégradable peut être :

- un polymère d'origine naturelle choisi dans le groupe comprenant la fibrine, le chitosane, le collagène, l'alginate, l'acide hyaluronique, et un mélange de deux ou plusieurs de ceux-ci ;
- une matrice, par exemple une matrice extracellulaire décellularisée ;
- un polymère synthétique choisi parmi les polyesters aliphatiques, notamment les polymères dérivés de l'acide lactique, notamment un poly(acide lactique), ou encore un poly(acide glycolique), un poly(acide lactique-co-acide glycolique), un poly-(acide malique), notamment le poly(beta-acide malique), un polycaprolactone, un polyglycérol sébacate, un polyéthylène glycol, un polyuréthane ou un poly(N-isopropylacrylamide), un mélange de deux ou plusieurs de ces polyesters aliphatiques, un co-polymère de polyesters aliphatiques ou un mélange de ces copolymères, ou
- un mélange d'un ou plusieurs polymère(s) d'origine naturelle choisi(s) parmi ceux précités et d'un ou plusieurs polymère(s) synthétique(s) choisi(s) parmi ceux précités.

Un polymère biocompatible biodégradable est un polymère de fibrine.

Ces polymères présentent en outre les avantages d'être, en particulier vis-à-vis des tissus cardiaques, cytocompatibles, de permettre la restauration des contraintes physiologiques du tissu traité et une restauration correcte du tissu déficient.

Quel que soit le polymère désignés dans la présente, il est de préférence de qualité suffisante pour être implantables et/ou injectables dans un organisme humain ou animal, dans ou sur un tissu déficient ou possiblement déficient à traiter, en particulier un tissu cardiaque, sans présenter de risque tant du point de vue chimique que biologique, notamment de contamination microbienne, pour l'organisme.

Selon l'invention, le polymère biocompatible biodégradable est un polymère de fibrine, il présente une taille de pores comprise entre 50 μm et 200 μm. Cette taille de pore permet avantageusement une inclusion suffisante de vésicules, et une vitesse de dégradation du polymère conjuguée à une libération des vésicules compatible avec la vitesse de restauration du tissu et/ou de ses fonctions.

La fibrine, avantageuse en particulier pour la mise en oeuvre de la présente invention en vue du traitement de tissus cardiaques, est fabriquée naturellement lors du mécanisme de coagulation sanguine, est généralement obtenue en laboratoire par mélange de fibrinogène et de thrombine. La thrombine agit comme une sérine-protéase qui convertit le fibrinogène soluble en brins de fibrine insoluble. Selon l'invention, par « fibrine », on entend toute forme de fibrine. Des exemples non limitatifs de fibrine comprennent la fibrine I, la fibrine II et des BB fibrine ou mélange de ces fibrines. La fibrine, lorsqu'elle est formée, constitue un réseau de fibrilles séparées par des pores dont la taille peut être précisément contrôlée par l'ajustement des concentrations respectives de fibrinogène et de thrombine, comme présenté dans les exemples ci-dessous. La fibrine peut être sous forme monomère ou polymère, dans laquelle la forme polymère est de préférence réticulée ou partiellement réticulée. Pour la mise en oeuvre de la présente invention, elle présente l'avantage de pouvoir être injectée, avec polymérisation ou gélation secondaire *in situ* et/ou implantée ou déposée sous forme de pièce ou « patch » sur la zone pathologique du tissu à traiter, et de se dégrader naturellement une fois implantée. Des exemples de méthodes et compositions utilisant un monomère de fibrine pour préparer des polymères de fibrine utilisables pour la mise en oeuvre de la présente invention sont décrits par exemple, dans les documents US 5,750,657 **[3]**, US 5,770,194 **[4]**, US 5,773,418 **[5]** et US 5,804,428 **[6]** ou encore dans les documents Wong et al., « Fibrin-based biomaterials to deliver human growth factors », Thromb Haemost 2003;89:573-82 **[7]**; Spicer et al. Fibrin glue as a drug delivery system. J Control Release 2010;148:49-55 **[8]**; Whelan et al., « Fibrin as a delivery system in wound healing tissue engineering applications », J Control Release 2014;196:1-8 **[9]**; Ahmad et al. Fibrin matrices: « The versatile therapeutic delivery systems », International Journal of Biomolecules 2015;81:121-36 **[10]**. La vitesse de polymérisation du fibrinogène peut être contrôlée en jouant sur le pH, par exemple en utilisant des solutions tampon pH, et/ou par un ajustement précis du rapport des concentrations respectives de fibrinogène et de thrombine. Des exemples de produits disponibles dans le commerce permettant d'obtenir de la fibrine utilisable pour la mise en oeuvre de la présente invention sont de préférence de qualité suffisante pour être implantée et/ou injectée dans ou sur un tissu d'un organisme humain ou animal. Il peut s'agir par exemple d'EVICEL (marque déposée), de TISSUCOL KIT (marque déposée), d'ARTISS (marque déposée), de TISSEEL (marque déposée), VIVOSTAT (marque déposée). Les inventeurs ont montré par exemple qu'un rapport de concentrations fibrinogène/thrombine de 20mg/mL-4 U, la dose de thrombine étant distribuée de façon homogène sous la forme de goutelettes de 25-$\mu$L, était optimal pour obtenir en 5 à 10 minutes à 37°C la polymérisation du mélange et la vectorisation de cellules cardiaques progénitrices dérivées de cellules souches embryonnaires initialement mélangées à la solution de fibrinogène, comme rapporté dans les documents Bellamy et al., « Long-term functional benefits of human embryonic stem cell-derived cardiac progenitors embedded into a fibrin scaffold », J Heart Lung Transplant. 2015 Sep;34 :1198-207 **[11]**, et Pr. Philippe Menasché et al., « Towards a clinical use of human embryonic stem cell-derived cardiac progenitors: a translational expérience », Eur Heart J. 2015;36:743-50 **[12]**.

Le chitosane ou chitosan, est un polyoside composé de la distribution aléatoire de D-glucosamine liée en $\beta$- et de N-acétyl-D-glucosamine, obtenu par exemple par désacétylation de la chitine. Il est disponible dans le commerce avec une grande variété de masses moléculaires et présente un degré de désacétylation compris entre 70% et 90% en général. Le poids moléculaire du chitosane varie de 3,800 à 20,000 Da. Le chitosane a des caractéristiques structurelles similaires aux glycosaminoglycanes (GAG) et semble imiter leur fonction comme décrit dans le document T. Chandy et CP Sharma, « chitosane comme biomatériau », Biomat, Art. Cells, Art. Org, 18; 1-24 1990 **[13]**. Des exemples de chitosanes disponibles dans le commerce et utilisables pour la mise en oeuvre de la présente invention peuvent être par exemple celui du lot n°SLBG1673V de Sigma Aldrich, 190 kD, viscosité 20-300 cps, avec un degré d'acétylation de 75 à 85% ou encore les agents hémostatiques à base de chitosane actuellement en usage clinique (ChitoFlex (marque déposée), ChitoGauze (marque déposée) PRO.

Le collagène est du collagène médical provenant par exemple de jeunes cheptels bovins ou porcins ou d'animaux libres certifiés non ESB (Maladie de la vache folle). Les animaux de distributeurs sont de préférence originaires de troupeaux fermés ou de pays qui n'ont jamais eu de cas rapporté d'ESB tel que l'Australie, le Brésil et le Nouvelle-Zélande. Il existe de nombreux travaux démontrant qu'en fonction du procédé de fabrication, le collagène ou sa forme dénaturée, la gélatine, peuvent être utilisés comme des vecteurs efficaces de substances sous la forme d'une solution injectable, comme décrit dans le document Ladage et al., « Stimulating myocardial régénération with periostin Peptide in large mammals improves function post-myocardial infarction but increases myocardial fibrosis », PLoS One. 2013;8:e59656 **[14]** ou d'un patch qui peut prendre la forme d'une éponge hémostatique, comme Gelfoam (marque de commerce) comme décrit dans le document Polizzoti et al. « Intrapericardial delivery of gelfoam enables the targeted delivery of Periostin peptide after myocardial infarction by inducing fibrin clot formation », PLoS One, 2012 ;7 :e36788 **[15]** ou Morcuende et al., « Neuroprotective effects of NGF, BDNF, NT-3 and GDNF on axotomized extraocular motoneurons in neonatal rats », Neuroscience. 2013;250:31-48 **[16]**, ou d'une membrane, comme décrit dans les documents Wei et al., « Epicardial FSTL1 reconstitution regenerates the adult mammalian heart », Nature. 2015 Sep 24;525(7570):479-85 **[17]** ; Jo et al., « Sequential delivery of BMP-2 and BMP-7 for bone régénération using a heparinized collagen membrane », Int J Oral Maxillofac Surg. 2015;44:921-8.) **[18]** ; et Oliver R. et al, 1976, Clin.. Orthop 115: 291-30; 1980, Rr J. Exp Chemin 61,544-549; 1981, Conn Tissue Res 9: 59-62 **[19]**.

L'alginate est un polysaccharide hydrophile anionique naturel abondamment produit dans la nature, comme décrit dans le document Skjak-Braerk, G.; Grasdalen, H.; Smidsrod, O. Inhomogeneous polysaccharide ionic gels. Carbohydr. Polym. 1989, 10, 31-54 **[20].** Des exemples d'alginates utilisables pour la mise en oeuvre de la présente invention sont décrits par exemple dans le document Skjak-Braerk et al. **[20],** ou encore dans les documents : Kolambkar et al., « An alginate-based hybrid system for growth factor delivery in the functional repair of large bone defects », Biomaterials 2011 ;32 :65-74 **[21]** ; Ruvinov et al., « The effects of controlled HGF delivery from an affinity-binding alginate biomaterial on angiogenesis and blood perfusion in a hindlimb ischemia model », Biomaterials 2010 ; 31 :4573-4582 **[22],** ainsi que dans le rapport de l'essai clinique ayant évalué une solution injectable d'alginate (Algisyl-LVR (marque déposée) rapporté dans le document Mann et al., « One-year follow-up results from AUGMENT-HF: a multicentre randomized controlled clinicaltrial of the efficacy of left ventricular augmentation with Algisyl in the treatment of heart failure », Eur J Heart Fail. 2016;18:314-25 **[23].**

L'acide hyaluronique est un glycosaminoglycane réparti largement parmi les tissus conjonctifs, épithéliaux et nerveux, par exemple, dans l'humeur vitrée et le liquide synovial. C'est l'un des principaux composants de la matrice extracellulaire. Pour la mise en oeuvre de la présente invention, on peut par exemple utiliser un produit décrit dans les documents : Silva et al., « Delivery of LLKKK18 loaded into self-assembling hyaluronic acid nanogel for tuberculosis treatment », J Control Release. 2016 Aug 10;235:112-24 **[24]** ou Soranno et al., « Delivery of interleukin-10 via injectable hydrogels improves renal outcomes and reduces systemic inflammation following ischémie acute kidney injury in mice » Am J Physiol Renal Physiol. 2016;311 :F362-72 **[25]** ou Eckhouse et al., « Local hydrogel release of recombinant TIMP-3 atténuâtes adverse left ventricular remodeling after experimental myocardial infarction », Sci Transl Med. 2014;6:223ra21 **[26].** Il peut s'agir par exemple aussi d'un produit destiné à la réparation cutanée, par exemple Hyalomatrix (marque déposée), comme décrit dans le document Landi et al., « Hyaluronic acid scaffold for skin defects in congenital syndactyly release surgery: a novel technique based on the regenerative model », J Hand Surg Eur Vol. 2014 Nov;39(9):994-1000 **[27]** ou d'un produit de comblement utilisé en chirurgie esthétique, comme ceux présentés dans le document Gutowski KA., « Hyaluronic acid fillers: Science and clinical uses », Clin Plast Surg. 2016;43:489-96 **[28].** L'acide hyaluronique présente l'avantage de pouvoir être injectable dans le tissu cardiaque en utilisant un cathéter, ou d'être délivré via une opération chirurgicale pour former un implant sous forme de gel, ce gel permettant une délivrance contrôlée des vésicules sur une période par exemple de 8 à 10 jours.

L'utilisation d'une matrice extracellulaire décellularisée est décrite. La matrice extracellulaire est une entité structurelle complexe entourant et supportant les cellules dans les tissus des mammifères. Elle se compose de trois grandes classes de biomolécules : les protéines structurales, par exemple, le collagène et élastine, des protéines spécialisées, par exemple, la fibrilline, la fibronectine et laminine, et des protéoglycanes, par exemple, les glycosaminoglycanes. De préférence, la matrice extracellulaire décellularisée est préparée de sorte que la structure de la matrice extracellulaire soit maintenue après avoir été décellularisée. On peut par exemple utiliser le protocole décrit dans le document WO2007025233 **[29]** ou dans la publication de Khorramirouz R et al., « Effect of three decellularisation protocols on the mechanical behaviour and structural properties of sheep aortic valve conduits », Adv Med Sci. 2014;59:299-307 **[30]** ou de He. et al., « Optimizationof SDS exposure on préservation of ECM characteristics in whole organ decellularization of rat kidneys », J Biomed Mater Res B Appl Biomater. 2016 Apr 8. doi: 10.1002/jbm.b.33668 **[31]** pour préparer une telle matrice, par exemple à partir d'un tissu qui de préférence est un tissu correspondant au, ou compatible avec, le tissu déficient qui doit être traité avec le biomatériau de la présente invention. La matrice peut être préparée à partir d'un tissu allogénique ou xénogénique. Il peut s'agir par exemple de tissus cardiaques, telle la matrice extracellulaire décellularisée issue de coeurs de porcs Ventrigel (marque de commerce) décrite par exemple dans le document Seif-Naraghi et al. « Safety and efficacy of an injectable extracellular matrix hydrogel for treating myocardial infarction », Sci Transl Med 2013 ;5:173ra25 **[32].**

Un polymère synthétique, par exemple choisi parmi les polyesters aliphatiques, notamment les polymères dérivés de l'acide lactique, notamment un poly(acide lactique) ou « PLA », ou encore un poly(acide glycolique), un poly(acide glycolique), un poly(acide lactique-co-acide glycolique), un poly-(acide malique), un polycaprolactone, un mélange de deux ou plusieurs de ces polyesters aliphatiques, un co-polymère de polyesters aliphatiques ou un mélange de ces copolymères est décrit.

Le PLA est un polymère entièrement biodégradable dans lequel les longues molécules filiformes sont construites par réaction d'un groupement acide d'une molécule d'acide lactique sur le groupement hydroxyle d'une autre pour donner une jonction ester. Dans le corps, une fois implanté, la réaction se fait en sens inverse et l'acide lactique ainsi libéré est incorporé dans le processus métabolique normal. On peut augmenter sa vitesse de biodégradation dans l'organisme, par exemple en stérilisant le polymère par une méthode agressive, en introduisant dans le polymère des fonctions acides ou des substances hydrophiles. Il est utilisé également en chirurgie où les sutures sont réalisées avec des polymères biodégradables qui sont décomposés par réaction avec l'eau ou sous l'action d'enzymes. C'est un matériau utilisable également par certaines imprimantes 3D. Deux principales méthodes de synthèse sont utilisées pour obtenir le PLA : la polycondensation ou la polymérisation par ouverture de cycle. Deux monomères sont utilisés: l'acide (L)-lactique (LLA) et l'acide (D)-lactique (DLLA). Des PLA sont par exemple décrits dans les documents Mohiti-Asli et al; Ibuprofen loaded PLA nanofibrous scaffolds increase prolifération of human skin cells in vitro and promote healing of full thickness incision wounds in vivo. J Biomed Mater Res B Appl Biomater. 2015 Oct 28. doi: 10.1002/jbm.b.33520 **[33]** ; Tyler et al., « Polylactic acid (PLA) controlled delivery carriers for biomedical applications », Adv Drug Deliv Rev. 2016 Jul 15. pii: S0169-409X(16)30211-3. doi: 10.1016/j.addr.2016.06.018 **[34]** ; et James et al., « Poly(lactic acid) for delivery of bioactive macromolecules » Adv Drug Deliv Rev. 2016 Jun 25. pii: S0169-409X(16)30193-4. doi: 10.1016/j.addr.2016.06.009 **[35]** ou par exemple disponible commercialement sous la marque Resomer (marque déposée - société EVONIK).

Des poly(acide lactique-co-acide glycolique) sont par exemple décrits dans les documents Liu et al. « HB-EGF embedded in PGA/PLLA scaffolds via subcritical CO2 augments the production of tissue engineered intestine. Biomaterials », 2016;103:150-9 **[36]** ; Thatcher et al ; « Thymosin β4 sustained release from poly(lactide-co-glycolide) microspheres: synthesis and implications for treatment of myocardial ischemia », Ann N Y Acad Sci. 2012;1270:112-9 **[37] ;** ou disponible commercialement par exemple sous la marque Resomer (marque déposée).

Des poly-(acide malique) sont par exemple décrits dans les documents Portilla-Arias JA et al., « Synthesis, degradability, and drug releasing properties of methyl esters of fungal poly(beta,L-malic acid) », Macromol Biosci. 2008;8:540-50 **[38]** ; Loyer et al., « Natural and synthetic poly(malic acid)-based dérivâtes: a family of versatile biopolymers for the design of drug nanocarriers » J Drug Target. 2014;22:556-75 **[39].**

Des polycaprolactones sont par exemple décrits dans les documents ; Patel JJ, et al., « Dual delivery of EPO and BMP2 from a novel modular poly-ε-caprolactone construct to increase the bone formation in prefabricated bone flaps », Tissue Eng Part C Methods. 2015;21:889-97 **[40]** ; Singh S, et al. The enhancement of VEGF-mediated angiogenesis by polycaprolactone scaffolds with surface cross-linked heparin. Biomaterials 2011 ;32 :2059-2069 **[41] ;** Jeong et al., « Three-dimensional polycaprolactone scaffold-conjugated bone morphogenetic protein-2 promotes cartilage régénération from primary chondrocytes in vitro and in vivo without accelerated endochondral ossification », J Biomed Mater Res A. 2012;100:2088-96 **[42]** ; ou sont ceux par exemple en cours d'évaluation clinique sous la forme d'une matrice électro-filée extemporanément pour le traitement des plaies cutanées, sous la référence NCT02680106, société Nicast Ltd..

Un mélange d'un ou plusieurs polymère(s) d'origine naturelle choisi(s) parmi ceux précités et d'un ou plusieurs polymère(s) synthétique(s) choisi(s) parmi ceux précités est décrit.

Par « vésicules extracellulaires de cellules souches », également appelées dans la présente « vésicules extracellulaires dérivées de cellules souches » ou « vésicules » ou « microvésicules », on entend l'un ou plusieurs des éléments produits par les cellules souches, notamment en culture *in vitro,* incluant les vésicules ou microvésicules, les exosomes, les corps apoptotiques et les microparticules qui se trouvent dans l'environnement dans lequel les cellules souches sont maintenues en vie. Les vésicules au sens de la présente invention comprennent donc une population hétérogène, dont les éléments sont différenciables notamment en fonction de leur taille et de leur contenu.

Agissant en véritables navettes, ces vésicules jouent un rôle essentiel dans la communication intercellulaire en assurant notamment le transfert de micro-ARN agissant sur certaines voies clefs de la signalisation, mais aussi de biolipides actifs et de protéines. Les inventeurs de la présente ont notamment validé l'équivalence fonctionnelle de cellules progénitrices cardiaques greffées dans le myocarde et des vésicules qui en sont issues. L'utilisation efficace de ces vésicules grâce au biomatériau de la présente invention permet d'assurer la présence des vésicules dans le tissu cible pendant une période suffisamment longue pour qu'elles puissent exercer leurs effets et d'éviter qu'une élimination rapide ne compromette l'efficacité de cette approche tout en provoquant une dissémination systémique non souhaitée.

Selon l'invention, les vésicules extracellulaires de cellules souches proviennent de cellules souches pluripotentes, en particulier de cellules induites à la pluripotence, quelque soit l'origine somatique ou multipotentes ou de cellules progénitrices cardiaques. Il peut s'agir par exemple de vésicules provenant de cellules choisies parmi des cellules pluripotentes, c'est-à-dire les cellules souches embryonnaires ou des cellules somatiques induites à la pluripotence ou cellules souches pluripotentes induites (iPS pour « induced pluripotent cells »), quelque soit l'origine somatique, c'est-à-dire des cellules prélevées chez l'adulte et reprogrammées en cellules pluripotentes par différents procédés incluant, mais non limités à, des adénovirus, des plasmides, des transposons, des virus de Sendaï, des ARNm synthétiques et des protéines recombinantes, par exemple telles que décrites dans le document Takahashi et Yamanaka, « A decade of transcription factor-mediated reprogramming to pluripotency. », Nat Rev Mol Cell Biol. 2016;17:183-93 **[43].**

Il peut s'agir par exemple de vésicules provenant de cellules choisies parmi des cellules multipotentes, par exemple des cellules souches mésenchymateuses, et tout particulièrement de cellules choisies parmi des cellules cardiaques, vasculaires, musculaires, rétiniennes, neurales, médullaires, ostéo-cartilagineuses, hépatiques, rénales, intestinales, hématopoïétiques, et du système immunitaire, et tout particulièrement, mais pas exclusivement, de cellules dendritiques.

Le choix des cellules, et par voie de conséquence des vésicules qui sont utilisées pour la mise en oeuvre de la présente invention, est bien sûr dépendant de la cible thérapeutique visée lors de l'utilisation du biomatériau de la présente invention, en particulier du tissu déficient à réparer.

Selon l'invention, la préparation de vésicules utilisables pour la mise en oeuvre de la présente invention, à partir des cellules précitées présente l'avantage de mieux standardiser le produit final et d'aboutir à une préparation disponible à la demande. En effet, les vésicules sont sécrétées par les cellules en question dans leur milieu de culture dont on a préalablement vérifié qu'il ne contient pas (ou très peu) de vésicules qui représenteraient naturellement un facteur confondant. Les vésicules extracellulaires sont prélevées de ces milieux conditionnés. Les documents suivants décrivent des procédés de culture de cellules souches et la préparation de vésicules issues de ces cultures utilisables pour la mise en oeuvre de la présente invention, à partir de différents types cellulaires :

- Anaïs Kervadec et al. **[2].**
- Barile et al., « Extracellular vesicles from human cardiac progenitor cells inhibit cardiomyocyte apoptosis and improve cardiac function aftermyocardial infarction », Cardiovasc Res 2014;103:530-41 **[44].**
- Bobis-Wozowicz et al., « Human induced pluripotent stem cell-derived microvesicles transmit RNAs and proteins to récipient mature heart cells modulating cell fate and behavior », Stem Cells. 2015;33:2748-61 **[45].**
- Lai et al. Exosome secreted by MSC reduces myocardial ischemia/reperfusion injury. Stem cell Res 2012;4:214-22 **[46].**
- Baulch et al. Cranial grafting of stem cell-derived microvesicles improves cognition and reduces neuropathology in the irradiated brain. Proc Natl Acad Sci USA. 2016;113:4836-41 **[47].**
- Nojima H et. al - Hepatocyte exosomes médiate liver repair and régénération via sphingosine-1-phosphate - J Hepatol. 2016 Jan;64(1):60-8 **[48].**
- Herrera MB et. al - Human liver stem cell-derived microvesicles accelerate hepatic régénération in hepatectomized rats - J Celle Mol Med. 2010 Jun;14(GB):1605-18 **[49].**
- Besse B et. al - Dendritic cell-derived exosomes as maintenance immunotherapy after first line chemotherapy in NSCLC - Oncoimmunology. 2015 Aug 12;5(4):e1071008. eCollection 2016 **[50].**
- Alessandra Fierabracci et. al - Recent Advances in Mesenchymal Stem Cell Immunomodulation: The Role of Microvesicles - Cell Transplantation, Vol. 24, pp. 133-149, 2015 **[51].**
- Mirjam C. Boelens et. al - Exosome Transfer from Stromal to Breast Cancer Cells Regulates Therapy Résistance Pathways - EMD Millipore Volume 159, Issue 3, p499-513, 23 October 2014 Amnis Imaging Flow Cytometry Integrating flow cytometry and microscopy **[52].**
- Paul D. Robbins et. al - Regulation of Immune Responses by Extracellular Vesicles - Nat Rev Immunol. 2014 March ; 14(3): 195-208 **[53].**
- Bin Zhang et. al - Mesenchymal Stem Cells Secrete Immunologically Active Exosomes - Stem Cells and Development, Volume 23, Number 11, 2014 Mary Ann Liebert, Inc. **[54].**
- L Kordelas et. al - MSC-derived exosomes: a novel tool to treat therapy-refractory graft-versus-host disease - Leukemia 2014;28;970 - 3 **[55].**
- Budoni M et. al - The immunosuppressive effect of mesenchymal stromal cells on B lymphocytes is mediated by membrane vesicles - Cell Transplant. 2013;22(2):369-79 **[56].**
- Christianson HC et. al - Cancer cell exosomes dépend on cell-surface heparin sulfate proteoglycans for their internalization and functional activity - Proc Natl Acad Sci USA. 2013 Oct 22;110(43):17380-5 **[57].**
- Ratajczak J et. al - Embryonic stem cell-derived microvesicles reprogram hematopoietic progenitors: évidence for horizontal transfer of mRNA and protein delivery - Leukemia. 2006 May;20(5):847-56 **[58].**
- Morelli AE et. al - The immune regulatory effect of apoptotic cells and exosomes on dendritic cells: its impact on transplantation - Am J Transplant. 2006 Feb;6(2):254-61 **[59].**
- Pêche H et. al - Présentation of donor major histocompatibility complex antigens by bone marrow dendritic cell-derived exosomes modulates allograft rejection - Transplantation. 2003 Nov 27;76(10):1503-10 **[60].**
- Celine Akyurekli et. al - A Systematic Review of Preclinical Studies on the Therapeutic Potential of Mesenchymal Stromal Cell-Derived Microvesicles - Stem Cell Rev and Rep, DOI 10.1007/s12015-014-9545-9, 05 August 2014 **[61].**
- Sarah E et. al - Neutrophil-derived microvesicles enter cartilage and protect the joint in inflammatory arthritis - Science Translational Medicine 25 November 2015 Vol 7 Issue 315 315ra190 **[62].**
- Bin Zhang et. al - HucMSC-exosome Mediated - Wnt4 Signaling is Required for Cutaneous Wound Healing - Stem Cells 2014;00:00-00 AlphaMed Press 2014 **[63].**
- Rekha Nair et. al - Extracellular Vesicles Derived from Preosteoblasts Influence Embryonic Stem Cell Différentiation - Stem Cells and Development Volume 23, Number 14, 2014 Mary Ann Liebert, Inc. **[64].**
- Vincenzo Cantaluppi et. al - Microvesicles Derived From Endothelial Progenitor Cells Enhance Neoangiogenesis of Human Pancreatic Islets - Cell Transplantation. Vol. 21. Pp. 1305-1320. 2012 **[65].**
- Hae Kyung et. al - Mesenchymal stem cells deliver exogenous miRNAs to neural cells and induce their différentiation and glutamate transporter expression - Delivery of miRNAs by MSCs to neural cells. [66]. Stem Cells Dev. 2014;23:2851-61.
- Hongqi Xin et. al - Systemic administration of exosomes released from mesenchymal stromal cells promote functional recovery and neurovascular plasticity after stroke in rats - Journal of Cerebral Blood Flow & Metabolism (2013), 1-5 2013 ISCBFM **[67].**

- Jian-ying Chen et. al - Therapeutic effects of mesenchymal stem cell-derived microvesicles on pulmonary arterial hypertension in rats-Acta Pharmacologica Sinica (4 August 2014) **[68].**
- Lee C et. al - Exosomes médiate the cytoprotective action of mesenchymal stromal cells on hypoxia-induced pulmonary hypertension - Circulation. 2012 Nov 27;126(22):2601-11 **[69].**
- Islam MN - Mitochondrial transfer from bone-marrow-derived stromal cells to pulmonary alveoli protects against acute lung injury - Mitochondria to the rescue, [Nat Med. 2012] **[70].**
- Changjin Lee et. al - Exosomes Médiate the Cytoprotective Action of Mesenchymal Stromal Cells on Hypoxia-Induced Pulmonary Hypertension - Circulationaha.112.114173 Published online before print October 31, 2012 **[71].**
- Aliotta JM et. al - Stable cell fate changes in marrow cells induced by lung-derived microvesicles - J Extracell Vesicles. 2012 Apr 16;1 **[72].**
- Der-Cherng Targ et. al - Induced Pluripotent Stem Cell-Derived Conditioned Medium Attenuates Acute Kidney Injury by Downregulating the Oxidative Stress-Related Pathway in Ischemia-Reperfusion Rats - Cell Transplantation, Vol. 25, pp. 517-530, 2016 **[73].**
- Bruno S et. al - Microvesicles derived from mesenchymal stem cells enhance survival in a lethal model of acute kidney injury - PloS One. 2012;7(3):e33115 **[74].**
- Cristina Grange et. al - Biodistribution of mesenchymal stem cell-derived extracellular vesicles in a model of acute kidney injury monitored by optical imaging - International Journal of Molecular Medicine 33: 1055-1063, 2014 **[75].**
- Grange C et. al - Biodistribution of mesenchymal stem cell-derived extracellular vesicles in a model of acute kidney injury monitored by optical imaging - Int J Mol Med. 2014 May;33(5):1055-63 **[76].**
- Cantaluppi V et. al - Microvesicles derived from endothelial progenitor cells protect the kidney from ischemia-reperfusion injury by microRNA-dépendent reprogramming of résident renal cells - Kidney Int. 2012 Aug;82(4):412-27 **[77].**
- Vincenzo Cantaluppi et. al - Microvesicles derived from endothelial progenitor cells protect the kidney from ischemia-reperfusion injury by microRNA-dependent reprogramming of résident renal cells - Kidney International advance online publication 11 April 2012 **[78].**
- Joseph V. Bonventre et. al - Microvesicles from Mesenchymal Stromal Cells Protect against Acute Kidney Injury - J Am Soc Nephrol 20: 925-932, 2009 **[79].**
- Stefania Bruno et. al - Mesenchymal Stem Cell-Derived Microvesicles Protect Against Acute Tubular Injury - J Am Soc Nephrol 20: 1053-1067, 2009 **[80].**
- Janet E. et. al - Cranial grafting of stem cell-derived microvesicles improves cognition and reduces neuropathology in the irradiated brain - PNAS Early Edition - www.pnas.og/cgi/doi/10.1073/pnas.1521668113 **[81].**
- Masako Nakano et. al - Bone marrow-derived mesenchymal stem cells improve diabetes-induced cognitive impairment by exosome transfer into damaged neurons and astrocytes - Scientific reports/6:24805/ **[82].**

Ainsi, les cellules souches sont maintenues en vie dans le milieu de culture, *in vitro,* et les vésicules produites par celles-ci sont récupérées, avantageusement purifiées, par exemple comme décrit dans les documents précités et/ou soumises à une filtration tangentielle et/ou une chromatographie, pour ensuite mettre en oeuvre la présente invention.

Selon l'invention, le polymère biocompatible inclut les vésicules extracellulaires de cellules souches. Par « inclut » on entend que le polymère est imprégné, à la manière d'une éponge, des vésicules ou que les vésicules sont mélangées avec le polymère en phase liquide et sont piégées dans la structure du polymère au cours de sa polymérisation. La solution proposée par la présente invention peut ainsi être considérée comme une fonctionnalisation ou inclusion du polymère biocompatible choisi avec les vésicules choisies pour permettre une libération contrôlée de ces dernières et éviter un lavage rapide auquel exposerait la seule injection des vésicules en milieu aqueux. Cette inclusion ou fonctionnalisation peut être réalisée notamment grâce au choix judicieux des polymères précités, à la nature des vésicules choisies et à la technique utilisée pour inclure les vésicules dans le polymère.

Des procédés d'inclusion conformes à la présente invention utilisables pour sa mise en oeuvre sont décrits ci-dessous. Cette inclusion est de préférence réalisée en quantité suffisante pour permettre au biomatériau de la présente invention de relarguer les vésicules tout au long de sa dégradation, et ainsi de maintenir, de manière prolongée, le traitement du tissu déficient par le biomatériau. La limite supérieure d'inclusion est notamment celle liée à la capacité d'inclusion du polymère choisi. Egalement, il est utile que l'inclusion soit suffisante pour assurer le traitement prolongé du tissu déficient.

Le procédé de l'invention est simple et économique. Il peut être mis en oeuvre à partir du polymère choisi ou à partir des monomères précurseurs du polymère choisi, partiellement polymérisés ou non, et des vésicules choisies.

Le biomatériau selon l'invention est obtenu par un procédé comprenant une étape de trempage du polymère biocompatible biodégradable dans un milieu liquide biocompatible comprenant des vésicules extracellulaires de cellules souches pluripotentes ou multipotentes ou de cellules progénitrices cardiaques, ou de mélange du polymère biocompatible biodégradable et/ou du ou des monomères correspondants, avec des vésicules extracellulaires de cellules souches ou progénitrices cardiaques.

La présente invention a également pour objet un procédé de fabrication d'un biomatériau selon l'invention comprend une étape de trempage du polymère biocompatible biodégradable dans un milieu liquide biocompatible comprenant des vésicules extracellulaires de cellules souches pluripotentes ou multipotentes ou de cellules progénitrices cardiaques ou par mélange du polymère biocompatible biodégradable ou du ou des monomères correspondants avec des vésicules extracellulaires de cellules souches pluripotentes ou multipotentes ou de cellules progénitrices cardiaques, lesdites vésicules étant un ou plusieurs éléments produits par lesdites cellules choisis parmi les microvésicules, les exosomes et les corps apoptotiques.

Selon l'invention, par « milieu liquide biocompatible » on entend un milieu liquide qui préserve les vésicules, tant dans leur forme que dans leur fonctionnalité. Il s'agit de préférence d'une solution aqueuse reproduisant suffisamment le milieu dans lequel les cellules souches produisent lesdites vésicules, *in vitro* ou *in vivo.* Il peut s'agir par exemple d'une solution proche ou identique au milieu de culture *in vitro* des cellules souches choisi pour produire les vésicules, ou d'une solution physiologique préservant les vésicules. Les milieux de culture cités dans les documents précités conviennent comme milieu liquide biocompatible. De préférence, la concentration des vésicules dans le milieu liquide est élevée, afin de permettre une inclusion élevée des vésicules dans le polymère pour constituer le biomatériau de la présente invention. Selon l'invention, de préférence, la concentration des vésicules est de $3\times10^{10}$ à $3\times10^{12}$ (30 milliards à 3000 millards) de vésicules par mL.

Afin d'atteindre une concentration préférée, par exemple à partir d'un milieu de culture liquide de cellules souches, il est possible de procéder à la concentration des vésicules par microfiltration ou par centrifugation du milieu de culture liquide des cellules souches. Des exemples de méthodologies utilisables pour obtenir les microvésicules dans un milieu liquide biocompatible sont décrites dans les documents **[2]** et **[44]** à **[82]** précités.

Selon l'invention, le polymère peut être fabriqué dans un premier temps, puis éventuellement séché ou partiellement séché de façon à absorber davantage de vésicules, à la manière d'une éponge, puis imprégné par les vésicules. Ainsi également, les monomères précurseurs du polymère choisi ou le polymère partiellement réticulé peu(ven)t être mélangé(s) avec les vésicules choisies, par exemple avec le milieu biocompatible comprenant les vésicules, avant polymérisation. Suivant le procédé de l'invention, on obtient alors un polymère incluant les vésicules.

Selon l'invention, le polymère, ou son précurseur monomère ou polymère partiel imprégné ou mélangé avec le milieu biocompatible comprenant les vésicules, peut donc être administré au patient sous forme injectable pour une polymérisation *in situ* dans le tissu ou zone pathologique à réparer, ou implanté sous forme de pièce ou « patch » déjà polymérisé(e) sur le tissu ou zone pathologique à réparer.

Selon l'invention, par « pièce » ou « patch », on entend une pièce de biomatériau selon l'invention, incluant des vésicules, qui peut être appliquée sur une zone *in vivo* à traiter, par exemple un tissu déficient, comme défini dans la présente. Cette pièce ou « patch » a de préférence une dimension qui permet de couvrir suffisamment, partiellement ou en totalité, la surface de la portion pathologique du tissu humain ou animal à traiter, et de préférence une épaisseur qui permet de contenir un nombre suffisant de vésicules pour le traitement afin de libérer de manière prolongée lesdites vésicules. Selon l'invention, la surface de la pièce ou « patch » qui peut être en contact avec le tissu à traiter peut avantageusement avoir une dimension de 5 à 30 $cm^2$, de préférence de 15 à 25 $cm^2$. Selon l'invention, l'épaisseur de la pièce ou « patch » qui peut être en contact avec le tissu à traiter peut avantageusement être de 0,5 à 2 mm, de préférence de 0,8 à 1,2 mm.

Par exemple, selon l'invention, avantageusement, une pièce ou un patch ayant des dimensions telles que celles précitées, peut comprendre avantageusement de $10^{10}$ à $10^{12}$ vésicules.

Selon l'invention, du monomère ou du polymère partiel tel que défini ci-dessus peut être utilisé comme « colle » pour implanter la pièce ou patch sur le tissu ou sur la zone pathologique à réparer, la polymérisation permettant ensuite l'immobilisation du patch sur le tissu. Selon l'invention, l'immobilisation du patch sur le tissu peut également être obtenue en collant le patch sur le tissu, soit en polymérisant à l'interface tissu-patch le monomère du polymère choisi, soit en utilisant par exemple une colle chirurgicale, par exemple l'un des polymères précités pouvant jouer ce rôle.

Le procédé de la présente invention peut, par exemple, également être mis en oeuvre par une technique permettant de définir précisément l'architecture tri-dimensionnelle du polymère, tels l'électro-filage ou l'impression 3D, par exemple au moyen de PLA, comme décrit ci-dessus. On peut utiliser par exemple la technique décrite dans le document Krishnan et al. « Engineering a growth factor embedded nanofiber matrix niche to promote vascularization for functional cardiac régénération » Biomaterials. 2016;97:176-95 **[83]** ; ou dans le document Lu Y, et al., « Coaxial electrospun fibers: applications in drug delivery and tissue engineering », Wiley Interdiscip Rev Nanomed Nanobiotechnol. 2016 Feb 5. doi: 10.1002/wnan.1391. [Epub ahead of print] **[84]** ; ou dans le document Gaetani R, et al., « Epicardial application of cardiac progenitor cells in a 3D-printed gelatin/hyaluronic acid patch préserves cardiac function after myocardial infarction », Biomaterials. 2015;61:339-48 **[85].**

Le biomatériau de la présente invention permet de traiter un tissu déficient sans greffe de cellules souches ou en complément d'une greffe de cellules souches.

Un biomatériau tel que défini dans la présente, pour une utilisation comme médicament est décrit. Comme exposé dans la présente, il peut s'agir par exemple d'un médicament destiné au traitement d'un tissu déficient humain ou animal. Le médicament peut être destiné par exemple au traitement d'un tissu choisi parmi un tissu cardiaque, vasculaire, musculaire, oculaire, cérébral, médullaire, ostéo-cartilagineux, hépatique, rénal, intestinal, hématopoïétique ou immunitaire.

Parmi les nombreux avantages déjà cités de la présente invention, et ceux apparaissant à l'homme du métier, on peut également citer que le biomatériau de la présente invention permet une vectorisation efficace des vésicules délivrées de façon minimalement invasive, par exemple par cathétérisme, ou chirurgicale et de dupliquer ainsi l'effet protecteur des cellules souches par une thérapie a-cellulaire permettant de s'affranchir de nombre des problèmes de l'art antérieur, notamment de logistique, de coût et d'éthique, inhérents à la transplantation des cellules elles-mêmes. Le biomatériau peut également accompagner une greffe cellulaire, afin notamment de faciliter la prise de la greffe, diminuer les pertes, accompagner le traitement du tissu originel et l'intégration et la fonctionnalisation des cellules souches greffées.

D'autres caractéristiques et avantages de la présente invention pourront apparaître à l'homme du métier, notamment à la lumière des expérimentations et des figures présentées ci-dessous à titre illustratif et non limitatif.

## Brève description des figures

- La figure 1 représente des images de microscopie cryo-électronique révélant la présence des différents sous-types de VE
- La figure 2 représente trois analyses NTA de trois préparations indépendantes de VE montrant une bonne reproductibilité dans la distribution de tailles des particules isolées.
- La figure 3 représente un Western Blot confirmant la présence de marqueurs d'exosomes et permettant de montrer la présence de ce type vésiculaire dans la préparation de VE.
- La figure 4 représente des photos représentant des coupes histologiques de patchs de fibrine colorées à l'hématoxyline/éosine
- La figure 5 représente un patch de fibrine avec une épaisseur de 1 mm facilement maniable et qui peut se fixer sur l'épicarde
- La figure 6 représente l'effet de la concentration en F/T sur l'élasticité du patch en kPa
- La figure 7 représente des photographies de patchs contrôles et des patchs contenant des VE
- La figure 8 représente une image d'ImageStream confirmant le relargage de VE, à partir du patch, de VE marquées à la calcéine.
- La figure 9 représente le test de cicatrisation montrant que les VE des iPS-Pg sont pro-angiogéniques
- La figure 10 représente les EV d'iPS-Pg améliorant la survie des cellules cardiaques en culture
- La figure 11 représente les résultats obtenus en ImageStream confirmant la présence de fluorescence uniquement dans les cellules incubées avec les VE préalablement marquées à la calcéine

## Exemples

### Exemple 1 : Procédé d'obtention de vésicules de cellules souches

A titre d'exemples non limitatifs, on utilise pour la production de vésicules destinées à la mise en oeuvre de la présente invention les protocoles suivants :

- Pour la production de vésicules de cellules cardiaques, le protocole décrit dans le document Barile et al. **[44].**
- Pour la production de vésicules de cellules souches mésenchymateuses, le protocole décrit dans le document Lai et al. **[46].**
- Pour la production de vésicules de cellules souches neurales, le protocole décrit dans le document Baulch et al. **[47].**

Par exemple dans le cas de vésicules extra-cellulaires (VE) de progéniteurs cardiovasculaires dérivés d'iPS, on a utilisé des cellules progénitrices cardiaques dérivées d'iPS humaines (iPS-Pg: iCell Cardiac Progenitor Cells, ref.: CPC-301-020-001-PT) qui proviennent du fournisseur Cellular Dynamics International (CDI, Madison, WI, USA). Ces progéniteurs issus d'iPS cryopréservés, sont décongelés et ensemencés à une densité cellulaire de 78 000 cellules/cm2, dans une flasque préalablement coatée à la fibronectine (ref.: 11051407001, Roche Applied Sciences, Indianapolis, IN, USA) et cultivés pendant 4 jours à 7% de CO2 et à 37°C dans du milieu sans sérum selon le protocole de prolifération recommandé par le fournisseur : William's E medium (ref.: A12176-01, Life Technologies, Saint Aubin, France) avec le Cocktail B du pack : « hepatocyte maintenance supplément pack » (ref.: CM4000, Life Technologies), 25µg/mL de gentamicine (ref.: 15750, Life Technologies) et 1µg/ml de FGF-2 de Zebrafish (ref.: GFZ1 Cell Guidance System, Cambridge, UK). Les VE sont produites et sécrétées par des cellules cultivées en milieu sans sérum. Les milieux sont changés au deuxième jour de culture (J2) et le milieu conditionné est collecté au quatrième jour de culture (J4). Ce milieu conditionné contient des VE sécrétées pendant 48H (jour 3 et jour 4). Il est récupéré pour l'isolation des VE. Les milieux et conditions de cultures pourraient être modifiés en fonction du type cellulaire, par exemple mésenchymateuses ou neurales.

Quelles que soient les cellules souches utilisées, pour l'isolement des VE, les milieux conditionnés sont récupérés et soumis à une centrifugation classique de 1200g pendant 6 min pour culoter les cellules et débris cellulaires contaminants. Le surnageant ainsi clarifié est transféré dans un tube propre. Il peut être congelé à -80°C pour le stocker ou utilisé frais pour l'isolation des VE. Pour l'isolation, le milieu clarifié est ultracentrifugé dans des tubes d'ultracentrifugation stériles à 100 000 g pendant 16 h, sous vide, à une température de 4°C avec une accélération et décélération maximale. Le surnageant après ultracentrifugation est éliminé et le culot est resuspendu dans un petit volume de PBS stérile, filtré à 0,1um. La préparation de VE peut être utilisée immédiatement pour la mise en oeuvre de la présente invention ou conservée à -80°C.

Les vésicules obtenues sont caractérisées et analysées par microscopie cryo-électronique, par la technique « Nanoparticle Tracking Analysis » (NTA) et par Western blot.

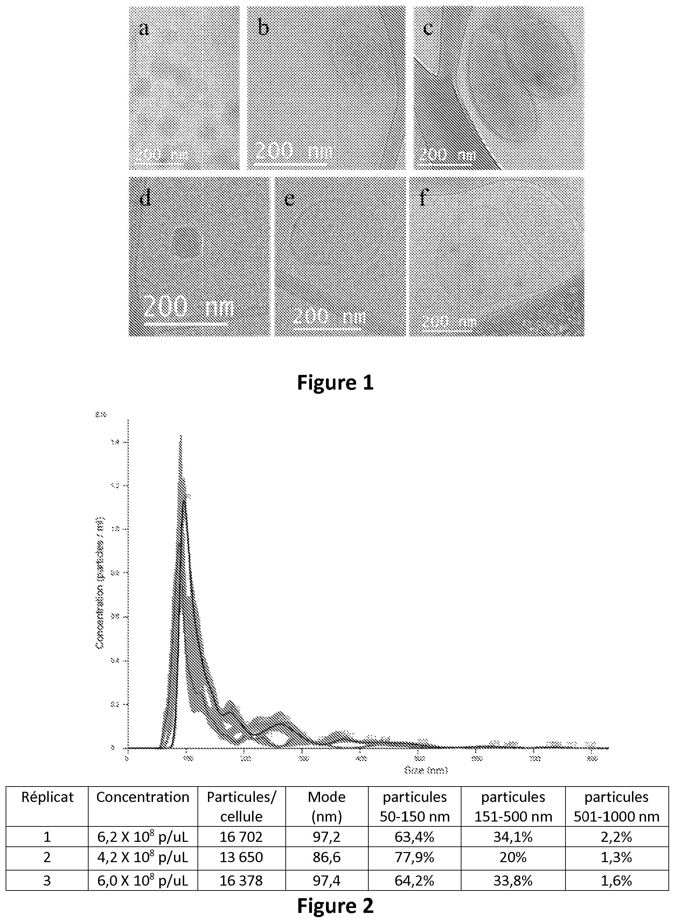

La microscopie cryo-électronique permet de visualiser les VE et d'identifier la présence des différentes sous-populations de VE, telles que les exosomes (50-150nm), les microparticules (100-500nm) et les corps apoptotiques (particules multivésiculaires >500 nm). La figure 1 annexée montre des images (a) à (f) de microscopie cryo-électronique révélant la présence des différents sous-types de VE dans le cas de VE issues de progéniteurs cardiovasculaires dérivés d'iPS. Les structures et la taille des exosomes (a, d) et des microparticules (b, e, c) sont visibles. Des structures multivésiculaires (f) qui pourraient être des corps apoptotiques sont aussi observées.

La technique « Nanoparticle Tracking Analysis » (NTA), sur une plate-forme Nanosight LM-10 (marque de commerce), avec laser 488nm, (NTA-3.2, Malvern Instruments Ltd., Malvern, UK) permet de déterminer la distribution des tailles de particules ainsi que la concentration de VE dans chaque préparation dans le cas de VE issues de progéniteurs cardiovasculaires dérivés d'iPS. La figure 2 annexée montre trois analyses NTA de trois préparations indépendantes de VE (replicats 1, 2 et 3) montrant une bonne reproductibilité dans la distribution de tailles des particules isolées.

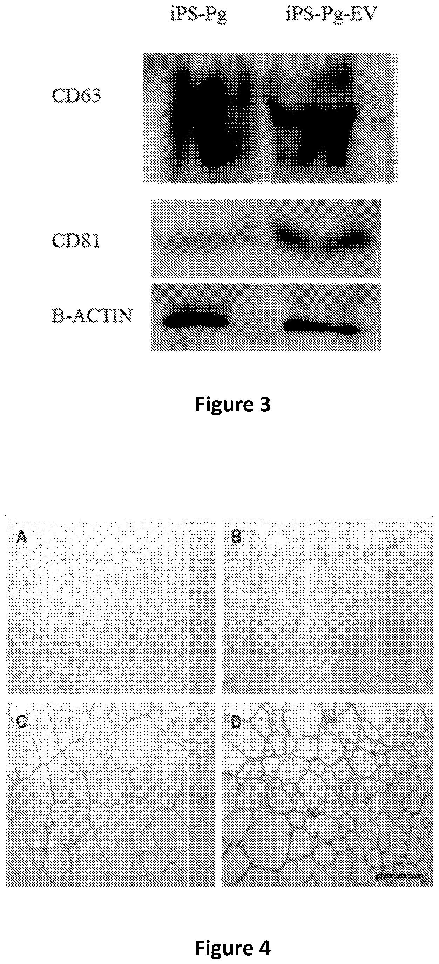

La technique Western blot permet d'analyser avec des anticorps, notamment CD81 et CD63 caractéristiques, la présence d'exosomes, sous-type de VE notamment dans le cas de VE issues de progéniteurs cardiovasculaires dérivés d'iPS. La figure 3 annexée montre le résultat de cette analyse confirmant la présence de marqueurs d'exosomes et permettant de montrer la présence de ce type vésiculaire dans la préparation de VE. Le CD81 est enrichi dans les vésicules par rapport à ces cellules sécrétrices.

**Exemple 2** : **Marquage des vésicules**

Dans cet exemple, l'une ou l'autre des deux techniques de marquage des vésicules détaillées ci-dessous sont utilisées, afin de vérifier l'intégration ou inclusion des vésicules dans le polymère choisi lors de la préparation du biomatériau de la présente invention, un marquage au moyen d'un colorant et un marquage avec de la calcéine-AM (AM = acétoxyméthyl) :

(a) Marquage au colorant

Pour marquer la membrane de tous les sous-types de VE obtenus, de manière indiscriminée, nous utilisons le colorant hydrophobe, DiD Vybrant cell tracer (marque de commerce) (réf : V-22887, VybrantTM Cell-Labeling Solutions, Molecular Probes), ci-après « DiD ». Les milieux conditionnés clarifiés (voir ci-dessus) sont transférés dans des tubes d'ultracentrifugation. Le volume est complété avec du PBS jusqu'à 15-20 mL. Un microlitre de DiD Vybrant cell tracer est rajouté par mL de milieu conditionné et homogénéisé. Les milieux conditionnés sont ultracentrifugés à 100 000g pendant 3h à 4°C. Les culots sont resuspendus dans du PBS. Le volume est encore complété à 20 mL et la suspension est encore ultracentrifugée à 100 000g pendant 90 min à 4°C pour enlever le DiD libre contaminant. Le culot résultant est resuspendu dans du PBS. Les vésicules ainsi marquées sont fluorescentes dans le rouge lointain.

(b) Marquage des VE avec la calcéine-AM

On utilise le protocole décrit dans le document Gray WD et al, Identification of therapeutic covariant microRNA clusters in hypoxia-treated cardiac progenitor cell exosomes using systems biology. Circ. Res. 2015 Jan16 ; 116(2) :255-63 **[86].** Le précurseur de la calcéine-AM est une molécule hydrophobe non-fluorescente qui passe à travers la bicouche lipidique des cellules et des VE. En présence d'estérases fonctionnelles, le précurseur est clivé, le rendant fluorescent et hydrophile, piégeant le signal à l'intérieur de la cellule. La Calcéine-AM (cat. C3100MP, Life Technologie) est dissoute dans du DMSO avec une concentration finale de 1mM. Le milieu conditionné issu de la culture des iPS-Pg est incubé avec 2 μM de calcein AM à 37°C pendant 1h. Les milieux sont ultracentrifugés durant 16h à 100 000 g. Le culot de VE marquées est resuspendu dans du PBS. Après avoir ajouté 17 mL de PBS frais, la suspension est de nouveau ultracentrifugée pendant 16h à 100 000 g pour laver le culot. Ce culot de VE lavées est resuspendu dans du PBS propre pour les expériences d'internalisation. Le surnageant issu du lavage est utilisé comme contrôle pour voir les contaminations possibles par de la calcéine libre. Les VE marquées à la calcéine fluorescent dans le vert, et sont visibles par ImageStream (ImageStream (marque déposée) Mark II Image Flow Cytometer, Merck Millipore), un équipement permettant la combinaison d'imagerie microscopique avec la cytométrie en flux.

**Exemple 3** : **Préparation de biomatériaux selon l'invention**

3.a) Production des patchs de fibrine :

Nous avons préparé comme exposé dans cet exemple des patchs de fibrine. Le principe est de resuspendre les vésicules extracellulaires obtenues après centrifugation (voir exemple 1 ci-dessus) dans une solution aqueuse de fibrinogène et ensuite de mélanger cette solution avec une solution de thrombine. La formation du polymère de fibrine se fait, alors, tout en enrobant et piégeant les VE.

Les pièces de fibrine ou « patchs » sont préparés à partir du produit EVICEL® (Omrix Biopharmaceutical-Ethicon Biosurgery, Belgium) à deux composants suivants, pour l'obtention de fibrine :

- Fibrinogène humaine (solution de 2ml à 50-90 mg/ml)
- Thrombine humaine (solution de 2ml à 800-1200 U/ml) Les patchs sont formés à partir de mélanges d'une solution de fibrinogène et d'une solution de thrombine chacune dissoute dans du PBS ou une solution d'alpha-MEM. Le ratio des composants est présenté dans le tableau I ci-dessous, et le ratio des volumes des deux solutions est fixé à 1:1. Les concentrations indiquées dans le tableau I ci-dessous (paragraphe d) sont préparées à partir des deux composants précités, afin de tester, notamment, différentes concentrations de chacun des composants et raffiner les caractéristiques physiques des patchs de fibrine telles que la maniabilité du patch, l'élasticité du patch, la taille des mailles et la capacité d'inclusion et de relargage des vésicules.

Sur les figures et dans la présente, « F » suivi d'un chiffre représente la concentration en fibrinogène, le chiffre exprimant cette concentration en mg/mL ; et « T », la concentration en thrombine, le chiffre suivant exprimant cette concentration en U/mL.

3.b) Caractéristiques des patchs de fibrine selon la concentration des composants.

Plusieurs patchs ont été produits en variant la concentration de fibrinogène/thrombine (F/T). Les inventeurs ont constaté que plus la concentration de F/T est élevée et plus le patch se polymérise rapidement. L'effet principal de ce phénomène est une variation dans la taille des mailles à l'intérieur du patch : plus elle se polymérise rapidement, plus les pores sont grands. Ceci est facilement observable sur des sections de patchs coupés au cryostat et colorés à l'Hématoxyline/Eosine, comme représenté sur la figure 4 annexée. Les fibres de fibrine apparaissent de couleur violet foncé.

Sur la figure 4 annexée, la photographie (A) représente un patch avec 10 mg/mL de fibrinogène et 2U/mL thrombine (F10T2); la photographie (B) représente un patch avec F 20 mg/mL + T 4U/ml (F20T4) ; la photographie (C) représente un patch avec F 40 mg/mL + T 8U/ml (F40T8). Toutes les photos sont prises au même grossissement. Barre d'échelle : 200μm.

Les photos présentées sur cette figure montrent que la taille des pores à l'intérieur du patch varie avec les concentrations respectives de fibrinogène (F) et thrombine (T), où les moins concentrés (A) et (B) ont des pores plus petits que les plus concentrés (C et D). Dans les pièces ou patchs fabriqués, on a observé une taille de pores allant de 50 μm à 200 μm (micromètres).

Une conséquence des variations dans la structure du patch est sa maniabilité. Les inventeurs ont évalué la maniabilité et la suturabilité des patchs des différentes concentrations de F/T.

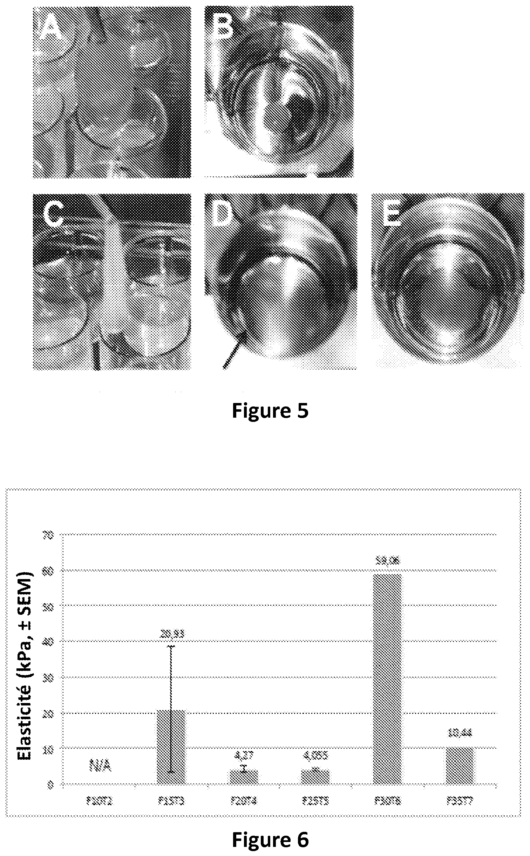

Les inventeurs ont observé une maniabilité du patch avantageuse pour l'imprégnation des VE et l'implantation entre F5T1 et F40T8. La maniabilité du patch est également fonction de son épaisseur. Les épaisseurs avantageuses notées au cours des expérimentations se situent de 0,8 à 1,2 mm. La figure 5 annexée présente à titre d'exemple l'un des patchs obtenus, celui-ci ayant une épaisseur de 1 mm.

3.d) L'élasticité :

L'élasticité des patchs a également été considérée par les inventeurs, selon la concentration en F/T. L'élasticité est directement mesurée à l'aide du système d'ultrason AirExplorerR (Supersonic Imagine) après avoir sélectionné la région à l'intérieur du gel. Les résultats, obtenus avec un dispositif Shear Wave Explorer, montrent une augmentation de la rigidité du patch avec l'augmentation des concentrations en F/T, comme représenté sur la figure 6 annexée. Sur cette figure est exprimé l'effet de la concentration en F/T sur l'élasticité du patch en kPa. Dans le cas d'un patch cardiaque, la fourchette d'élasticité optimale se situe entre 6 et 15 kPa avec une valeur préferentielle autour de 10 kPa. Toutefois, l'élasticité est à moduler en fonction de la nature du tissu cible.

3.e) Exemple de protocole d'inclusion des VE dans un patch de fibrine de la taille d'un puit d'une plaque 24 puits. :

**Matériels** :

- Agarose 5%, stérile
- VE des iPS-Pg préparé suivant l'exemple 1, repris dans du PBS stérile
- Solution de fibrinogène en PBS stérile (Evicel, Omrix Biopharmaceuticals N.V, Belgique)
- Solution de thrombine en PBS stérile (Evicel, Omrix Biopharmaceuticals N.V, Belgique)
- Milieu alpha-MEM (réf. : 22571020 GIBCO, Life Technologies)
- Plaque 24 puits (« P24 ») (réf : 009224, TPP, Dutscher)

**Méthode** :

1/ Préparer de l'agarose à 5%, à stériliser à l'autoclave (protocole liquide à 134°C).
2/ Déposer cette solution d'agarose encore liquide dans des puits P24 pour recouvrir le fond des puits et laisser durcir pendant 20 min. L'agarose empêche le patch de fibrine d'adhérer au fond du puit.
3/ Choisir les concentrations en fibrinogène et en thrombine en fonction du tableau ci-dessous pour avoir les caractéristiques physiques du patch désiré.
4/ Préparer dans du PBS ou alpha-MEM la solution A avec la concentration de fibrinogène désirée et mélangée avec $20\times10^6$ VE par patch de taille P24 dans un volume final de 150µL.
5/ Préparer dans du PBS ou alpha-MEM la solution B avec la concentration de thrombine désirée dans un volume final de 150µL par patch de taille P24.
6/ Bien mélanger les 150µL de la solution A avec les 150µL de la solution B en faisant des allers-retours avec la pipette pour bien homogénéiser et en évitant de faire des bulles.
7/ Laisser incuber 20-25 min à 37°C
8/ Rajouter du milieu alpha-MEM ou PBS pour recouvrir le patch et éviter le dessèchement. Il faut environ 1ml de liquide pour recouvrir un patch de taille d'un puit d'un plaque 24-puit.
9/ Les patchs sont laissés au repos, avantageusement 4 heures.

**Tableau 1 :** Concentrations de solutions de fibrinogène (F) et Thrombine (T) pour différentes pièces ou patchs de fibrine

| | **Concentration de fibrinogène (F) dans la solution A** | **Concentration de Thrombine (T) dans la solution B** |
|---|---|---|
| **F1T0,25** | 1 mg/ml | 0,25 U/ml |
| **F2.5T0,5** | 2,5 mg/ml | 0,5 U/ml |
| **F5T1** | 5 mg/ml | 1 U/ml |
| **F10T2** | 10 mg/ml | 2 U/ml |
| **F20T4** | 20 mg/ml | 4 U/ml |
| **F40T8** | 40 mg/ml | 8 U/ml |

3.f) Visualisation des VE marquées dans un patch de fibrine :

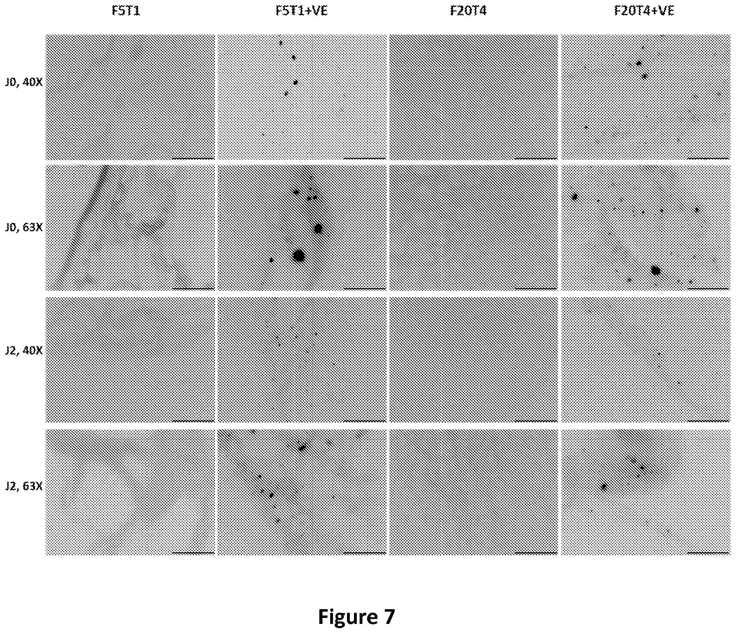

Les vésicules extracellulaires sont marquées à la calcéine AM et incluses dans le patch de fibrine. Ce patch est ensuite enrobé d'OCT (marque de commerce) (réf : TFM-5, MM France), congelé dans l'azote liquide et coupé au cryostat. L'épaisseur de coupe varie entre 7 et 10μm. Les coupes sont visualisées par microscopie à fluorescence. Elles sont représentées sur la figure 7, sous forme de photos prises au grossissement 40x ou 63x.

On appelle « patch contrôle » les patchs n'ayant pas reçu de vésicules marquées. On appelle « patch VE » les patchs contenant des vésicules extracellulaires marquées à la calcéine. La figure 7 montre le patch contenant ou ne contenant pas de vésicules extracellulaires marquées à la calcéine à deux temps différents de l'expérience (J0 : immédiatement après polymérisation ; J2 : après deux jours d'incubation à 37°C dans du milieu alpha-MEM) et à deux grossissements différents (40x et 63x) sur un microscope à fluorescence. Les lasers ont volontairement été poussés pour que nous puissions voir le patch pour les patchs contrôles.

Sur cette figure, les points noirs correspondent aux VE fluorescentes visibles à l'intérieur du patch.

Dans les patchs contrôles, il n'y a pas de fluorescence verte ponctiforme présente ce qui signifie qu'il n'y a pas de vésicules présentes. On peut voir dans les patchs contrôles et les patchs VE le maillage des patchs. Par contre dans les « patchs VE », on voit la présence de points verts dans les deux patchs aux différentes concentrations utilisées. La présence de ces points verts signifie qu'il y a la présence de vésicules extracellulaires marquées à la calcéine. Ces vésicules extracellulaires sont présentes à l'intérieur des patchs.

3.g) Etude de relargage des VE d'un patch de fibrine

Les patchs des différentes concentrations de F/T présentés ci-dessus (tableau I) contenant 20x $10^6$ VE marquées à la calcéine-AM sont incubés dans du milieu alpha-MEM pur, c'est-à-dire un milieu ne contenant aucune particule de la taille des VE, pendant 48 heures.

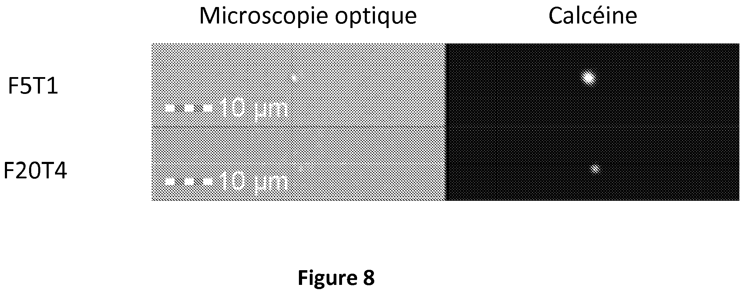

Ensuite, ces milieux sont collectés et centrifugés à 1200 g pendant 10 min pour culoter les débris de fibrine, et le surnageant est transféré dans un tube propre. Les milieux ainsi clarifiés sont analysés par ImageStream. Les résultats sont présentés sur la figure 8.

Dans les milieux conditionnés des conditions F5T1+VE et F20T4+VE, les vésicules extracellulaires ont été marquées et incluses dans les patchs. La présence de particules dans les milieux conditionnés traduit le relarguage des VE. Cette libération a été confirmée par ImageStream.

Les résultats obtenus à l'ImageStream montrent qu'il y a bien un relargage des vésicules extracellulaires dans le milieu conditionné après 48h. La comparaison des patchs F5T1+VE et F20T4+VE est faite par rapport au contrôle qui est le milieu récupéré des patchs F5T1 seul et F20T4 seul. Dans les milieux des conditions F5T1+VE et F20T4+VE, en comparaison avec les contôles négatifs, nous observons des événements positifs.

3.h) Production des patchs d'acide hyaluronique (ne faisant pas partie de l'invention) :

Le patch peut peut être celui décrit dans Eckhouse et al. [24]. Il peut s'agir en particulier du produit destiné à la réparation cutanée Hyalomatrix (marque déposée), ou du produit de comblement utilisé en chirurgie esthétique présentés dans le document Gutowski KA [25]. On utilise de préférence un milieu milieu alpha-MEM ou PBS pour l'imprégnation avec les vésicules, comme décrit ci-dessus.

Les inventeurs élaborent des formulations qui peuvent être injectées dans un tissu cardiaque en utilisant un cathéter (approche percutanée) ou administrées chirurgicalement (thoracotomie) pour former des implants en forme de gel libérant les VE pendant 8 à 10 jours à un taux contrôlé. L'HA non réticulé déjà utilisé pour des applications cliniques est sélectionné.

3.i) Production des patchs de collagène (ne faisant pas partie de l'invention) :

Le patch peut être une éponge hémostatique, comme Gelfoam (marque de commerce) comme décrit dans le document Polizzoti et al. [15] ou d'une membrane, comme décrit dans les documents Wei et al. **[16]**

**Exemple 4 : Test d'efficacité des vésicules extracellulaires de la présente invention pour la réparation de tissus déficients**

a) Analyses de la bio-activité des VE - Test de cicatrisation (« Wound healing assay »):

Des cellules endothéliales vasculaires issues de sang de cordon humain (HUVEC single donor, ref: C-12200, PromoCell, Heidelberg, Germany) sont ensemencées à une densité de 42 000 cellules/$cm^2$ sur des plaques 96 puits non coatées et cultivées dans du milieu complet pour cellules endothéliales (Endothelial Cell Growth Medium, ref: C-22210, PromoCell, Heidelberg, Germany plus Endothelial Cell Growth Medium Supplement Pack, ref :C-39210, PromoCell) à 37°C et à 5% $CO_2$ jusqu'à confluence (toute la nuit).

Un trou ou « zone de scratch » ou « blessure » est ensuite créé sur une couche confluente de cellules à T0 (Temps initial 0) en la grattant avec un cône de pipette de 10μl et la cicatrice est repérée en temps réel par un microscope inversé (Nikon-ECLIPSE Ti, NIKON CORPORATION, Tokyo, Japan).

Les cellules sont ensuite cultivées durant 24 heures dans différentes conditions qui sont les suivantes : soit dans du milieu complet (contrôle positif), soit dans le milieu « pauvre » (contrôle négatif; milieu sans complément), soit dans le milieu pauvre avec les VE isolées à partir de sérum de veau foetal pur, soit dans le milieu pauvre avec les VE d'iPS-Pg. Les images de chaque puits sont prises toutes les 6 heures durant 24 heures. La surface de la zone de scratch est déterminée à chaque temps par une analyse d'image.

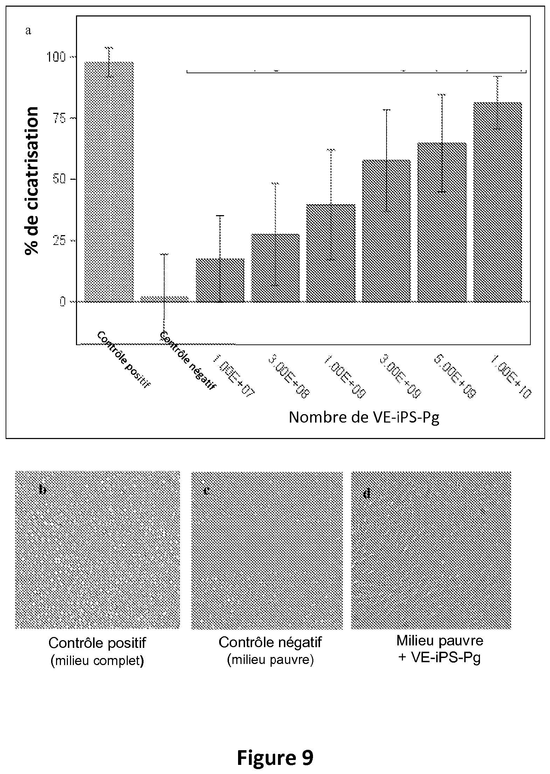

Le pourcentage de cicatrisation est calculé comme suit: % cicatrisation = 100% × (surface T0 - surface T) / (surface T0). Chaque condition est moyennée avec trois réplicats et testée au moins sur trois préparations indépendantes de VE (réplicats biologiques). Nous avons constaté que les VE stimulent la migration des cellules endothéliales de manière très reproductible et que le nombre de vésicules a un effet, comme réprésenté sur la figure 9 annexée. Sur cette figure, est représenté le test de cicatrisation montrant que les VE des iPS-Pg sont pro-angiogéniques :

(a) Histogramme représentant en ordonnée le pourcentage de cicatrisation après 18h d'incubation des HUVEC avec différentes quantités de VE. Un effet dose sur la migration des cellules est observé.
(b) La blessure est entièrement cicatrisée dans le contrôle positif, (c) pas cicatrisée dans le contrôle négatif, et (d) presque entièrement cicatrisée à forte dose de VE.

b) Analyses de la bio-activité des VE - test de viabilité:

Une lignée de cellules de rat immortalisées de myoblastes cardiaques, H9c2, est ensemencée sur plaques 6-puits préalablement coatées avec 0,2% de gélatine à une densité de 25 000 cellules/$cm^2$ et cultivées à 5% $CO_2$ et à 37°C dans du milieu complet: DMEM glutamax (ref.: 10566-016, GIBCO, Waltham, MA, USA), avec 10% de FBS et 1% de Penicilline/ Streptomycine/ Amphotericine (PSA). Après 24 ou 48h, les cellules issues de deux puits sont comptées (T0) avec le Muse (marque de commerce) Cell Analyzer, selon les directives du fournisseur.

Pour les autres puits, les milieux sont changés et les cellules sont incubées pendant 27h dans différentes conditions qui sont les suivantes : soit dans du milieu complet (contrôle positif), soit dans le milieu sans sérum (DMEM glutamax + 1% PSA) (contrôle négatif, milieu de stress), soit dans le milieu sans sérum avec les VE à différentes concentrations.

Après vingt-sept heures d'incubation, les cellules viables dans chaque condition sont comptées avec le Muse (marque de commerce) Cell Analyzer (T27). Chaque condition est évaluée en duplicat et les valeurs obtenues sont moyennées. Le pourcentage de viabilité est calculé comme suit: 100% × (T27 cell count-T0 cell count)/T0 cell count.

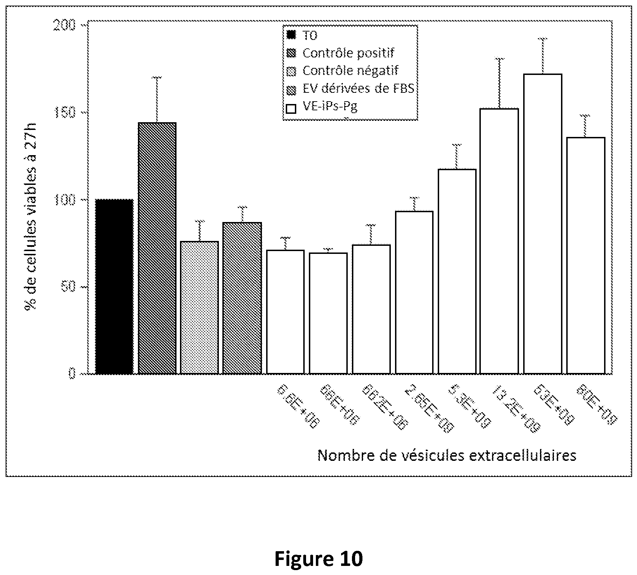

La figure 10 annexée montre les résultats de trois réplicats biologiques (c'est-à-dire trois différentes préparations de VE d'iPS-Pg évaluées par des expériences de test de viabilité indépendantes). Les EV améliorent la survie des cellules cardiaques.

c) Analyses de l'internalisation des VE par des cellules cibles :

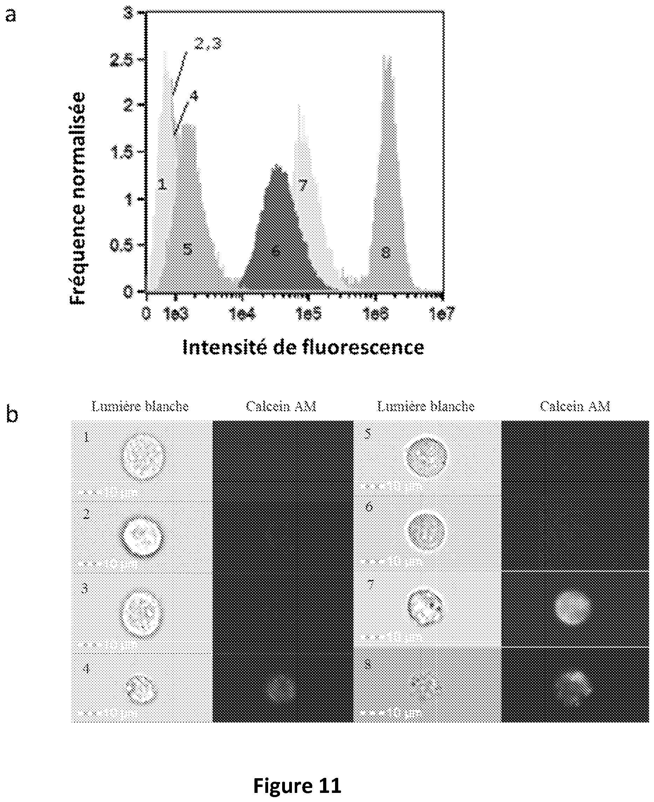

Le test de viabilité est utilisé pour déterminer si les VE marquées à la calcéine AM sont internalisées. Comme décrit ci-dessus, les H9c2 sont cultivées dans un milieu pauvre avec ou sans l'addition de VE. Ici, les VE sont préalablement marquées ou non à la calcéine. Après 20h d'incubation, les cellules sont collectées et analysées par l'ImageStream. La présence de fluorescence dans les cellules H9c2 indique une internalisation de VE marquées. Nous avons constaté que les cellules incubées avec les VE marquées sont fluorescentes, tandis que les contrôles ne le sont pas, comme cela est visible sur la figure 11 annexée.

Sur cette figure, les résultats obtenus en ImageStream confirment la présence de fluorescence uniquement dans les cellules incubées avec les VE préalablement marquées à la calcéine. Les histogrammes d'intensité de fluorescence et les images des cellules cultivées en présence de calcéine AM montrent une forte fluorescence verte dans ces cellules (a-8 et b-7 : contrôle positif, cellules + calcéine à T19h, c'est-à-dire, une heure de marquage ; a-7 et b-8 : cellules + calcéine pendant les 20 heures de l'expérience). Les contrôles négatifs sans calcéine sont bien négatifs (a-4 et b-1 : milieu complet ; a-3 et b-2 milieu pauvre ; a-2 et b-5 VE de iPS-Pg non-marquées ; a-1 et b-3 : VE de FBS non-marquées).

Les cellules incubées avec le surnageant après le premier lavage sont légèrement plus fluorescentes que les contrôles négatifs (a-5), mais cette fluorescence n'est pas visible par microscopie (b-6). Finalement, les cellules incubées avec les VE des iPS-Pg marquées à la calcéine sont vertes avec une intensité beaucoup plus forte qu'avec le surnageant de lavage et moindre que les cellules marquées directement avec la calcéine (a-6 et b-4).

d) Analyse de la réparation d'un tissu déficient par un biomatériau conforme à la présente invention

Des patchs contenant des vésicules ou non sont préparés selon le protocole décrit dans les exemples ci-dessus. Une fois fabriqués, ces patchs sont déposés ou fixés par suture ou colle sur le coeur déficient.

L'évaluation de l'efficacité des patchs de vésicules est réalisée par echochardiographie à différents temps pour suivre l'évolution de la fonction cardiaque et l'effet comparés au contrôle (c'est-à-dire au patch sans vésicules).

**Exemple 5** : **Préparation de collagène et inclusion du collagène avec des vésicules extracellulaires issues de cellules multi ou pluripotentes** (ne faisant pas partie de l'invention)

Nous avons considéré dans cet exemple le Gelfoam (marque déposée - Pfizer), qui est un collagène dénaturé ou gélatine à forte capacité d'absorption.

Avec le Gelfoam (marque déposée), des patchs maniables et suturables de bonnes élasticités sont obtenus. Le principe de ce patch est de l'incuber sous forme sèche avec une solution de vésicule obtenue comme décrit ci-dessus pour que celle-ci imprègne le patch avec les vésicules.

**Exemple 6 : Préparation de VE à partir de cellules endothéliales humaines de cordon ombilical**

1. Préparation des VE

Les cellules endothéliales humaines du cordon ombilical (HUVEC; C-12200 ; Promocell)) sont cultivées dans des flasques, coatées à la gélatine 0,2%, à une densité de 5000¢/cm2 dans un milieu de culture complet de cellules endothéliales (Endothélial Cell Growth Medium ; C-22010 ; Promocell) supplémenté par un complément (kit C- 39210 ; Pomocell) qui sera renouvelé à J1 et à J4. Lorsque les cellules sont confluentes, le milieu complet est remplacé par un milieu pauvre (milieu sans supplément). Deux jours après, le milieu conditionné est centrifugé à 1200g pendant 6 minutes pour éliminer les débris cellulaires avant de procéder à l'isolation des VE.

Les vésicules sont marquées avec deux types de marqueurs : le DiD (Dialkyl Indocarbocyine Dye) et la calcéine AM.

i.) Le Dialkyl Indocarbocyine Dye : Le marqueur Dialkyl Indocarbocyine (DiD) est un fluorophore lipophile qui est internalisé par les VE et émet une fluorescence dont la longueur d'onde en émission est de 665 nm. Les VE sont marquées au DiD (V-22887, Vybrant Cell-labeling Solution, Invitrogen) par addition directe de 1μl de ce marqueur dans 1 ml de milieu conditionné. Le milieu marqué est conservé à 37° C pendant une heure avant d'être ultra-centrifugé une première fois pour l'isolation des VE puis une seconde fois pour éliminer le marqueur qui n'a pas été internalisé. Le DiD a été utilisé pour marquer les VE de FBS car celles-ci ne se marquent pas à la calcéine AM.

ii.) La calcéine Acetoxy Methyl :La calcéine Acetoxy Methyl (AM) est un fluorophore hydrophobe qui traverse la membrane bi-lipidique. Une fois à l'intérieur des VE, il est clivé sous l'action d'une estérase qui rend la molécule hydrophile et fluorescente. Par ce mécanisme, la calcéine est un marqueur plus spécifique pour l'étude des VE que le DiD. La calcéine AM (cat. C3100MP, Life Technologies) est diluée dans 50 μl de DMSO pour obtenir une concentration initiale de 1 mM. Un volume de cette solution de calcéine AM est rajouté au milieu conditionné afin d'obtenir une concentration finale de 2 μM. Le milieu conditionné marqué est conservé pendant une heure à 37° C pour permettre le marquage des VE avant l'étape d'isolation.

Les milieux conditionnés des cellules ou le sérum de veau foetal (FBS), qui contient naturellement des VE, sont soumis à une ultracentrifugation à 37 500 rpm (100 000g) pendant 16 heures à 4° C. A la fin du cycle, le surnageant est enlevé et le culot est re-suspendu avec du PBS filtré à 0,1 μm. Quand les VE ont été préalablement marquées, 20 ml de PBS filtré est ajouté aux culots re-suspendus, et un deuxième cycle d'ultracentrifugation est lancé à 37 500 rpm pendant 16h à 4° C pour éliminer les fluorophores non internalisés par les VE.

2. Préparation des patchs

Le fibrinogène et la thrombine utilisés pour la fabrication de la fibrine proviennent du kit EVICEL (Omrix Biopharmaceuticals-Ethicon Biosurgery, Belgium). Pour la préparation des patchs, une plaque de 24 puits est préparée 1 heure avant le dépôt du mélange fibrinogène thrombine, en déposant 1ml d'agarose stérile au fond de chaque puits. Cette étape permet au patch formé d'adhérer seulement au niveau des parois du puits sans coller au fond pour faciliter sa récupération par la suite.

Les patchs sont formés par un mélange de fibrinogène et de thrombine à deux concentrations différentes selon l'expérience. Des patchs préparés avec 5mg/ml de fibrinogène et 1u/ml de thrombine (F5T1) et des patchs préparés avec 20mg/ml de fibrinogène et 4u/ml de thrombine (F20T4). Le fibrinogène et la thrombine sont dilués dans 3 types de solutions différentes, selon la condition, soit de l'alpha-minimal essential medium (αMEM), du PBS ou du Na Cl. Le fibrinogène et la thrombine sont mélangés dans la plaque de 24 puits, à part égale soit 150 μl chacun pour induire la polymérisation. Après 1 heure à 37°c, le temps que le patch polymérise, 1ml (ou plus) de milieu (le même que celui qui a été utilisé pour le patch) est ajouté par-dessus le patch pour éviter le dessèchement de celui-ci. L'inclusion des vésicules dans le patch se fait avant la polymérisation, en ajoutant les vésicules dans la solution de fibrinogène avant de les mélanger avec la thrombine.

3. Etude de la structure microscopique des patchs

Les patchs de fibrine sont récupérés des puits 4 heures après leur préparation, soit J0, et 3 jours après, soit J3. Les patchs récupérés sont disposés dans des cupules remplies avec une solution de cryo-préservation (OCT®) et sont congelés d'une manière progressive dans de l'azote liquide puis stockés à -80° C. Ces patchs sont coupés au cryostat en coupes de 10μm d'épaisseur, disposées sur une lame de microscopie qui sera congelée par la suite. Ces lames sont colorées à l'hématoxyline/éosine puis analysées au scanner de lames. L'analyse des images ainsi obtenues a été réalisée à l'aide du logiciel NDPView.

4. Mise en évidence des VE au niveau des patchs

a.) Microscope à fluorescence : des patchs contenant des VE de FBS marquées au DiD sont récupérés et congelés à J0 et J3. Après la découpe au cryostat, les lames sont montées au fluoroshield et étudiées au microscope à fluorescence Leica DM 2000 (Leica Wetzlar, Allemagne) couplé à une caméra Quicam CDD (Qimaging corp. Surrey BC canada). Les images ont été analysées à l'aide du logiciel Métamorph.

b.) Microscope confocal : le microscope confocal est un microscope optique capable de réaliser des images de très faible profondeur appelées coupes optiques. Le système confocal simple photon utilise une lumière d'excitation dont la longueur d'onde excite directement le fluorophore. Le système confocal est caractérisé par une fenêtre (ou iris confocal) placée devant le photo-détecteur qui élimine la fluorescence provenant des régions non focales. Ce système a été utilisé pour imager les VE au niveau du patch avec une très haute résolution. Pour cela, 3 types de patchs ont été préparés. Des patchs F5T1 contenant $21.10^9$ de VE d'HUVEC doublement marquées à la calcéine et au DiD, des patchs F5T1 avec des VE non marquées et des patchs F5T1 nus sans VE, pris comme contrôles négatifs. Ces patchs sont récupérés directement sur des lamelles adaptées au confocal avec du milieu de montage Dako® et ont été étudiées à l'aide d'un microscope confocal de la plateforme d'imagerie cellulaire de l'institut IMAGINE à l'hôpital Necker dirigée par Meriem Garfa-Traoré.

c.) Microscope bi-photons : La microscopie à excitation bi-photonique est un outil qui combine les techniques d'optique du microscope confocal avec une excitation multi-photonique qui utilise des lumières d'excitation dans l'infrarouge. Dans ce cas seul le point de focalisation du faisceau laser est excitateur. Du fait de l'excitation hautement localisée, le photo-bleaching des fluorophores ainsi que l'altération de l'échantillon sont réduits ce qui augmente la durée de l'expérience. L'utilisation d'une lumière d'excitation à une longueur d'onde élevée (>900nm) assure une plus grande pénétration à l'intérieur de l'échantillon (jusqu'à 500μm au lieu de 150μm) offrant la possibilité de travailler sur des échantillons plus épais. Le microscope bi-photons permet l'étude de la structure tridimensionnelle du patch en réalisant plusieurs coupes optiques sans devoir sectionner le patch et risquer de perdre du matériel. Trois patchs F5T1 ont été préparés pour cette étude. Deux patchs avec ou sans VE d'HUVEC doublement marquées au DiD et à la calcéine AM et un patch sans VE ont été récupérés sur des lamelles adaptées au confocal avec du milieu de montage type Dako® et étudiés à l'aide du microscope bi-photons de la plateforme d'imagerie cellulaire à l'institut IMAGINE de l'hôpital Necker.

5. Quantification des VE

a.) Nanoparticle Tracking Analysis : Après production et isolation des VE, celles-ci sont quantifiées à l'aide d'un Nanoparticle Tracking Analysis (NTA). Le NTA ou nanosight (LM 10 laser 488nm, Malvern Instruments Ltd, Malvern, UK) est une méthode d'analyse des nanoparticules permettant de les suivre individuellement. Cette technologie permet d'avoir une concentration et une courbe de distribution par la taille des particules par diffusion dynamique de la lumière et analyse de leur mouvement Brownien. Le NTA comporte un microscope optique permettant de détecter la lumière reflétée par les particules suspendues dans la solution contenue dans une chambre fermée.

b.) ImageStream : L'ImageStream (ImageStream®X Mark II Imaging Flow Cytometer, Amnis) (IS) est une technologie qui associe les propriétés d'analyse de la cytométrie en flux et l'imagerie microscopique.

Cette méthode d'analyse permet de détecter plus de 10 marqueurs fluorescents différents avec une haute résolution. Cette technique nécessite très peu de matériel (15μl) pour l'analyse ce qui la rend très avantageuse. Cette technique permet de visualiser les VE marquées et sert de contrôle lors du marquage des VE et elle permet aussi de mesurer la concentration des VE marquées.

6. Quantification du relargage des VE de FBS marquées au DiD

Des patchs de fibrine F5T1 contenant 21 $10^9$ de VE de FBS marquées au DiD ont été coulés dans une plaque 24 puits. Après 1 heure de polymérisation, 1 ml de milieu αMEM a été ajouté et la plaque est conservée dans l'incubateur à 37° C. Le patch et son milieu de suspension ont été récupérés à différents temps de contrôle, et congelés à -80° C.

Les milieux récupérés ont été filtrés à l'aide de tamis à 40 μm avant l'analyse à l'ImageStream, pour éviter l'obstruction du capillaire de l'appareil d'ImageStream lors de l'aspiration des milieux.

7. Quantification du relargage des VE d'HUVEC marquées à la calcéine AM

Des patchs de fibrine F5T1 contenant 21 $10^9$ de VE d'HUVEC marquées à la calcéine AM ont été coulés dans une plaque 24 puits. Une heure après l'ajout de la thrombine au fibrinogène, 1,5 ml de αMEM ont été ajoutés dans chaque puits. A chaque temps, les milieux ont été récupérés selon deux schémas différents :

- Changement de milieux : le milieu est récupéré en totalité, soit 1,5 ml, et est remplacé par un volume de milieu αMEM équivalent.
- Aliquot : Un aliquot de 250 μl est récupéré à chaque temps sans ajout de milieu supplémentaire.

Les échantillons sont récupérés à différents temps de contrôle et sont conservés soit à -80° C ou à 37° C jusqu'à leur analyse par la suite.

8. Quantification de la dégradation du pach

La quantification de la dégradation des patchs de fibrine se fait par quantification des D-Dimères par un kit ELISA D-Dimères (DHDDIMER, Human D-DIMER ELISA KIT, Thermo Scientific) selon le protocole du fournisseur. Le dosage des D-Dimères se fait sur les mêmes échantillons de milieux récupérés pour le dosage des VE marquées à la calcéine afin de comparer le profil de libération des VE à partir du patch avec la dégradation de la fibrine.

9. Tests de maniabilité des patchs

Les tests de maniabilité ont pour but de déterminer la composition du patch optimal qui peut être utilisé par le chirurgien sans difficultés lors de l'opération. Un patch optimal est un patch qui est facile à récupérer avec une pince classique, se décolle facilement des parois du puits sans se déchirer, ne se rétracte pas suite au décollement et qui est capable de reprendre sa forme initiale une fois déposé sur le tissu lors de l'opération ou dans une solution de conservation lors des tests. Trois paramètres ont été choisis pour la comparaison de la prise en main des patchs :

- La prise en pince.
- Le maintien de la forme circulaire après sortie du puits.
- La reprise de la forme initiale du patch dans la solution de cryoconservation.

Les patchs de fibrine ont été préparés avec deux concentrations différentes de fibrinogène et thrombine, F5T1 et F20T4. A ces deux composants, fibrinogène et thrombine, un volume adéquat de milieu est rajouté. Trois milieux différents ont été testés lors de cette expérience, l'αMEM, le PBS et le Na Cl. Ces milieux ont été choisis pour leur utilisation courante en clinique. La préparation des patchs montre que ceux-ci polymérisent et se figent à des temps différents selon la concentration et le milieu ajouté. En effet, les patchs F20T4 polymérisent plus rapidement que les patchs F5T1, et pour une même concentration, les patchs préparés avec du milieu αMEM ou PBS durcissent plus rapidement que les patchs préparés avec le Na Cl. Quatre heures après le mélange du fibrinogène et de la thrombine dans les puits, les patchs sont récupérés et mis dans des petites cupules avec un gel de cryo-préservation des tissus (OCT®) qui permettra leur congélation.

Les patchs de fibrine, F5T1 et F20T4, préparés avec de l'αMEM sont faciles à récupérer et à déposer dans les cupules, gardent bien leur forme circulaire sans se replier sur eux-mêmes. Le patch F20T4 préparé avec du PBS présente les mêmes caractéristiques que celles obtenues avec les patchs mélangés avec l'αMEM, tandis que les patchs F5T1 préparés avec du PBS sont plus friables lors du décollement des parois, présentent une légère rétractation lors de la sortie du puits, mais qui disparait après étalement dans le gel de cryo-préservation.

**Tableau I : résultats des tests de maniabilité des patchs**

| Concentrations | Milieux | Prise en pince | Maintien de la forme | Ré-étalement |
|---|---|---|---|---|
| F5T1 | αMEM | + | + | + |
| | PBS | + | - | + |
| | Na Cl | - | - | - |
| F20T4 | αMEM | + | + | + |
| | PBS | + | + | + |
| | Na Cl | - | - | - |
| + : résultat positif (résistance prise en pince, maintien de la forme, ré-étalement)<br>- : résultat négatif (résistance prise en pince réduite, maintien de la forme réduite, ré-étalement difficile) | | | | |

Les patchs F5T1 contenant la solution NaCl n'ont pas réussi à bien polymériser. Lors de la récupération de ces patchs à J0, la texture n'était pas encore rigidifiée et collait à la pince lors de la sortie du puits. Un jour après leur préparation, ces patchs se sont rompus dans les puits de la plaque.

A l'issu de ces tests, nous avons conclu que les patchs préparés avec l'αMEM présentent les meilleurs résultats au niveau de la maniabilité, en particulier pour le maintien de la structure à la sortie du puits et l'absence de rétractation du polymère grâce à la rigidité de la texture obtenue. Ces caractéristiques rendent ce patch le meilleur candidat pour l'utilisation en clinique. Les patchs préparés avec le PBS montrent également de bon résultats.

10. Etude de la structure microscopique des patchs

Les patchs de fibrine sont destinés à véhiculer des VE qui présentent une taille de l'ordre du nanomètre. La structure microscopique de ces patchs a été étudiée afin de comparer les différentes architectures obtenues avec chaque condition de préparation et pour déterminer la condition optimale qui pourrait s'accorder avec l'addition des VE. Les différents patchs ont été congelés à différents temps après leur polymérisation. Les coupes obtenues au cryostat ont été colorées à l'hématoxyline/éosine et numérisés avec un scanner de lames.

Les résultats montrent qu'à J0 les patchs F5T1 préparés avec le αMEM ou le PBS, ne sont pas totalement polymérisés. En effet, l'étude de la structure microscopique de ces patchs montre une texture de la fibrine non réticulée comparé à la structure que l'on visualise, pour les mêmes conditions de concentration et de milieu, 3 jours après. A J3 les patchs F5T1 préparés avec le αMEM ou le PBS présentent une architecture bien structurée, avec des fibres fines et des petits pores comparés à ceux obtenus avec une forte concentration de fibrinogène.

Les patchs F20T4, préparés avec le αMEM ou le PBS présentent une structure similaire à J0 et à J3. Ces patchs polymérisent rapidement, dès l'ajout de la thrombine au fibrinogène, et s'organisent dès lors en un réseau de fibres avec des pores nettement plus grands que ceux visualisés avec de faibles concentrations. Les résultats montrent que des patchs obtenus avec la solution NaCl présentent une structure des fibres qui diffère de celle obtenue avec les deux autres milieux. A J0 le patch F5T1 préparé avec le NaCl présente des fibres très épaisses et de très grands pores non homogènes tandis que le patch F20T4, préparé avec la même solution, présente une structure comparable à celle observée avec les patchs F5T1 mélangés à l'αMEM ou le PBS à J0, donc une texture non polymérisée. A l'issu de ces résultats, il semble que les patchs préparés avec de faibles concentrations de fibrinogène, soit F5T1, sont les plus adéquat pour véhiculer les VE.

La petite taille des pores que forment les patchs F5T1 après polymérisation semble être plus adaptée à la taille des particules que nous souhaitons y inclure lorsqu'elles sont mélangées avec la fibrine. De plus, vu leur faible concentration, les patchs F5T1 se dégradent plus rapidement et devraient donc faciliter la libération de leur contenu une fois greffés au niveau du myocarde. La comparaison de ces résultats avec ceux obtenus lors des tests de maniabilité mènent à conclure que les patchs F5T1 mélangés à l'αMEM semblent représenter la combinaison le plus avantageuse pour l'inclusion des VE.

11. Mise en évidence des VE au niveau des patchs

Le but de cette étude a été de mettre en évidence la présence des VE dans le patch polymérisé après leur mélange au polymère en phase liquide et de voir comment ces particules s'organisent à l'intérieur d'un patch de fibrine en sachant que même si ces résultats ne sont pas nécessairement extrapolables en l'état à d'autres biomatériaux, ils fournissent un référentiel utile pour des mises au point ultérieures avec des polymères différents.

a.) Microscope à fluorescence : l'étude des patchs au microscope à fluorescence, montre que la fibrine émet une fluorescence spontanée dans le bleu. Plusieurs points rouges, de taille hétérogène, sont visualisés dans le patch uniquement lorsque des VE marquées au DiD y ont été incluses. Ces points sont visualisés uniquement avec le filtre rouge et ne sont pas visualisés dans les patchs contrôles où seul le DiD a été rajouté ou dans les patchs nus sans marquage. Ces points rouges pourraient correspondre aux VE de FBS marquées au DiD. Ces particules sont localisées préférentiellement sur les fibres de fibrine et sont rarement visualisées au niveau des pores du patch, peut-être à cause de la rupture de ces pores lors de la coupe des patchs au cryostat ce qui entraine une perte de matériel.

b.) Microscope confocal : des patchs F5T1 contenant des VE d'HUVEC doublement marquées au DiD et à la calcéine AM ont été analysés, au microscope confocal, en entier sans devoir les couper au cryostat. Le marquage des VE a été contrôlé par ImageStream avant leur inclusion dans le patch. L'étude du patch contenant les VE doublement marquées montre la présence de plusieurs points fluorescents dont la taille et la couleur évoquent les VE incluses. Ces particules arrondies sont retrouvées disséminées dans tout le patch. Certaines particules ne sont visualisées que dans le filtre vert, d'autres n'apparaissent que dans le filtre rouge et quelques particules semblent avoir internalisé les deux marqueurs et apparaissent dans les deux filtres, vert et rouge. Ces points fluorescents ne sont pas retrouvés dans les patchs contenant les VE non marquées et dans les patchs sans VE.

c.) Microscope bi- photons : les patchs F5T1 avec des VE doublement marquées à la calcéine AM et au DiD ou sans VE ont été analysés au microscope bi-photon. Le microscope bi-photons permet l'étude du patch sur toute son épaisseur, sans devoir le sectionner au cryostat ou marquer la fibrine par un fluophore. Les résultats d'analyse du patch au microscope bi-photons, montrent que la fibrine est spontanément fluorescente dans le filtre vert et le filtre rouge, ce qui permet de distinguer le réseau que forme la fibrine après sa polymérisation. L'étude du patch F5T1 contenant les VE marquées à la calcéine et au DiD, montre la présence de plusieurs points fluorescents, en vert et en rouge, de tailles différentes dispersées dans tout le patch évoquant les VE marquées. Les particules de grande taille pourraient correspondre à plusieurs vésicules disposées en amas dans le polymère. L'analyse du patch en z-stack, montre que ces particules se retrouvent, en majorité au niveau des fibres du réseau et rarement dans les cavités du patch. Ces particules fluorescentes ne sont pas retrouvées dans les patchs contrôles, avec VE non marquées et sans VE, où seul le réseau de fibrine est visualisé.

12. Quantification du relargage des VE à partir des patchs

Pour les expériences de relargage des VE, les patchs F5T1 αMEM ont été testés, car les patchs obtenus à cette concentration sont moins épais les patchs F20T4, donc ce choix de concentration peut entrainer une meilleure diffusion des VE du réseau de fibrine vers le milieu et une vitesse de dégradation des patchs plus rapide en particulier dans les conditions in-vitro.

a.) Quantification du relargage des VE FBS marquées au DiD

Les différents milieux sont récupérés à des temps différents selon le protocole et ont été conservés à -80° C. Le jour de l'analyse, tous les milieux ont été filtrés à l'aide de tamis de 40 μm pour éliminer les gros débris issus de la dégradation de la fibrine qui pourraient boucher le capillaire de l'appareil d'IS lors de l'aspiration des milieux. Pour cette expérience, les contrôles positifs sont :

- qVE DiD+ : est un échantillon contenant l'équivalent de la dose de VE marquées au DiD incluse dans un patch de fibrine soit $21.10^9$ de particules quantifiées au NTA (qVE NTA) suspendues dans 50 μl de PBS filtré à 0,1μm soit une concentration de 4,2.108 particules/μl ([VE]NTA)
- PBS DiD+ : est un échantillon de PBS filtré 0,1μm marqué avec un volume de DiD équivalent à la dose nécessaire pour un marquage de VE.
- αMEM DiD+ : est un échantillon de αMEM marqué avec un volume de DiD équivalent à la dose nécessaire pour un marquage de VE.

Les contrôles négatifs sont :

- qVE DiD- : est un échantillon contenant 21 $10^9$ de VE non marquées.
- PBS filtré : est un échantillon contenant 50 µl de PBS filtré à 0,1µm.

**Tableau II : Résultats par ImageStream des conditions contrôles** :

| Echantillons | | Objets/ml DiD+&SSC- |
|---|---|---|
| Contrôles positifs | q VE DiD+ $21\times10^9$ | 1.01E+08 ($[VE]_{IS}$) |
| | PBS DiD+ | 1,99E+06 |
| | MEM DiD+ | 1,64E+06 |
| Contrôles négatifs | q VE DiD- $21\times10^9$ | 3,36E+02 |
| | PBS filtré | 1,22E+03 |
| | alphaMEM | 6,17E+02 |

**Tableau III : Résultats de la quantification par IS des VE dans les milieux de relargage des patchs.**

| Echantillons | Temps | Objets/ml | qEV DiD+ | Rendement |
|---|---|---|---|---|
| F5T1+VE | J0 | 4,41E+05 | 4,41E+05 | 9% |
| F5T1+VE | J03 | 1,53E+05 | 1,53E+05 | 3% |
| F5T1+VE | J07 | 1,45E+05 | 1,45E+05 | 3% |
| F5T1+VE | J15 | 1,37E+05 | 1,37E+05 | 3% |

Les résultats obtenus à l'IS des échantillons de milieux de relargage des patchs sont résumés dans le tableau III. Le rendement de chaque condition est calculé par rapport au rendement de l'IS pour 21 $10^9$ VE. Ce chiffre équivaut à $5,07\times10^6$ et il a été calculé comme suit :
Le rendement à 100% par IS :

$$([VE]IS / [VE]NTA) \times qVE\ NTA$$

$$\text{Soit } (1,01\times10^5 / 4,2\times10^8) \times 21\times10^9$$

Les résultats obtenus à l'IS montrent que le patch F5T1 libère une certaine quantité de VE à J0 (environs 10% de ce qui a été inclus dans le patch). De J0 à J3, une diminution de 2/3 de la quantité de VE libérées est observée et le rendement des patchs passe de 9% à 3%. De J3 à J15 la quantité de VE dans le milieu de relargage est restée constante. Cette diminution des VE observée de J0 à J3, peut être due au changement de conformation du patch qui passe d'un état semi polymérisé à J0 à un état complètement polymérisé et structuré dans les jours qui suivent, ce qui pourrait emprisonner plus de particules dans le polymère durant cette période par une force électrostatique qui attirerait les VE libres dans le milieu vers l'intérieur du patch. Pour cette analyse, tous les milieux ont été filtrés à 40 µm; cette étape de filtration qui a semblé nécessaire pour la bonne utilisation de la machine pourrait présenter un biais pour la quantification des VE suite à une perte d'une certaine quantité de particules qui pourraient rester piégées dans les débris de fibrine éliminées par le tamis. L'analyse des milieux PBS et αMEM contenant du DiD à l'IS montre que ce marqueur se présente sous une forme de particules suspendues comparable à celle visualisée avec les VE marquées au DiD, ce qui présente un deuxième biais lors de la quantification du nombre d'événements positifs en comptabilisant les particules de DiD comme étant des VE libérées dans le milieu. Pour cette raison le choix du marqueur pour le reste des expériences de quantification des VE s'est porté sur la calcéine AM.

**b.)** Quantification du relargage des VE d'HUVEC marquées à la calcéine AM

Pour cette expérience, plusieurs conditions ont été expérimentées selon la méthode de récupération des milieux, changement de milieu ou aliquots, et température de conservation des échantillons, soit à -80° C ou à 37° C. Le changement de milieu des patchs a pour but d'accélérer le phénomène de diffusion des particules du patch vers le milieu et la dégradation de la fibrine. La conservation des échantillons à 37°C est choisie pour permettre la poursuite du phénomène de dégradation des débris de fibrine récupérés dans les tubes afin de libérer un maximum de particules qui pourraient rester piégées à l'intérieur; cela permettrait de s'affranchir du biais de quantification des VE lié à l'étape de filtration des échantillons avant leur analyse à l'IS,

La comparaison des pourcentages de libération des VE par le patch, dans les quatre conditions testées, montre des profils assez similaires, avec un pic de relargage durant les 3 premiers jours, après la préparation du patch, puis une libération faible de particules dans le temps. Ce pic correspond à la présence d'une certaine quantité de particules dans les milieux récupérés à J3, quantifiées entre 10 et 20% des VE incluses dans le patch, selon la condition. Ce pic retrouvé à J3 pourrait correspondre à des VE suspendues dans le milieu $\alpha$MEM qui n'ont pas été totalement incorporées dans le patch lors de sa polymérisation et se sont retrouvées en suspension lors de l'ajout du milieu. Puis la quantité de VE libérées par le patch, dans les jours suivants est en moyenne de 3% à 5%. Cette différence dans la quantité des VE libérées par les patchs entre les trois premiers jours et les jours suivants, pourrait être liée à la transformation du polymère durant cette période, qui passe d'une forme non réticulée, qui laisse diffuser facilement les particules à une forme polymérisée qui renferme les VE piégées dans les fibres néoformées. Dans la condition où le milieu a été aliquoté et conservé à 37° C, la quantité de VE retrouvées à J3 est moins importante que celle retrouvéedans les trois autres conditions; cela pourrait être dû à une polymérisation plus rapide de ce patch ou à la présence de moins de VE lors de la préparation de celui-ci. La libération des VE dans les jours suivants est légèrement plus importante dans les conditions où le milieu a été récupéré en totalité et changé par du milieu vierge. Cette différence de relargage pourrait être la conséquence du phénomène de diffusion passive des particules vers le milieu vierge ajouté, ou une dégradation plus importante du polymère dans ces conditions.

La température de conservation des échantillons n'influence pas la quantité de VE trouvées dans ces milieux, donc la conservation à 37° C ne semble pas entrainer une libération plus importante des particules qui seraient piégées dans les débris de fibrine récupérés lors des contrôles.

La comparaison des profils de libération cumulée des VE à partir des patchs selon la condition avec la courbe de libération passive théorique montre que le relargage des VE est très faible et ne suit pas l'évolution de cette courbe, donc la libération des particules à partir du polymère ne semble pas être liée à la diffusion passive de celles-ci.

13. Monitorage de la dégradation des patchs

Les mêmes milieux qui ont été analysés par IS pour quantifier la libération des VE ont été utilisés pour le dosage des D-Dimères. Les D-Dimères sont des produits issus de la dégradation de la fibrine et leur dosage sert de repère pour déterminer si la libération des VE à partir des patchs est bien dépendante de la dégradation du polymère ou pas. Le dosage des D-Dimères montre un pic à J3 dans toutes les conditions sauf dans l'échantillon récupéré par aliquot et conservé à 37° C. On note que le taux des D-Dimères évolue différemment dans cette condition, avec absence de pic à J3 et augmentation progressive des produits de dégradation de la fibrine au cours du temps. La comparaison de cette courbe avec le dosage des VE dans la même condition, montre un profil d'évolution similaire. La quantité de VE dans le milieu suit le taux de dégradation de ce patch et l'existence d'un pic de libération des VE à J3 moins important dans cette condition que dans les autres échantillons testés semble être liée à la texture du patch qui présente un niveau de dégradation moins important durant cette période que les autres patchs. Dans les autres conditions, l'évolution des D-Dimères est comparable entre elles. On retrouve un taux élevé à J3, ce qui correspond à une dégradation accrue de ces patchs, puis la quantité des D-Dimères chute. Cela pourrait correspondre au moment où le patch change de conformation suite à sa polymérisation et à son durcissement.

Dans les conditions où le milieu a été changé, le taux des D-Dimères diminue jusqu'à s'annuler, ce qui évoque une dégradation très faible ou inexistante de ces patchs. Donc le rendement des patchs en VE, légèrement plus important, dans ces conditions est lié à une meilleure diffusion des particules vers le milieu vierge ajouté. La comparaison des courbes de dosage de D-Dimères avec les courbes des dosages de VE dans chaque condition, montre une corrélation entre la dégradation du polymère et la quantité des particules retrouvées dans ces milieux. Un pic de libération des VE est retrouvé dans les 3 conditions où un fort taux de d-dimères a été dosé. A partir de J7, La concentration des VE est beaucoup moins importante dans les conditions où le milieu $\alpha$MEM a été changé, donc celles où la dégradation de la fibrine est presque inexistante.

**Exemple 7** : **Tests in vivo**

Dans cet exemple, le modèle porcin d'infarctus myocardique reperfusé est séléctionné pour plusieurs raisons: l'anatomie de l'artère coronaire de cette espèce et la forme du thorax le rend plus approprié pour les évaluations échocardiographiques que les moutons et le rapport taille cardiaque / poids corporel est proche de ceux de celui de l'homme. Le protocole inclut l'introduction transfémorale d'un cathéter d'angioplastie pour une visualisation initiale du réseau de l'artère coronaire suivie de l'insertion d'un fil guide permettant le placement d'un ballon d'angioplastie dans la partie médiane de l'artère coronaire descendante antérieure (LAD). Le ballonnet est ensuite gonflé progressivement pendant 90 minutes pour interrompre le flux sanguin dans la zone distale de la LAD, en aval de la deuxième branche diagonale majeure, confirmée par fluoroscopie. Après la déflation du ballon, une deuxième angiographie est effectuée pour confirmer la perméabilité du vaisseau. La prévention des arythmies peut être assurée par un prétraitement oral quotidien avec amiodarone et des bêtabloquants et une perfusion intra-procédurale d'amiodarone. En post-opératoire, les animaux peuvent recevoir une dose quotidienne d'aspirine et de clopidogrel. Deux séries d'expériences devraient être réalisées pour imiter les deux principales situations cliniques.

Trois semaines après la création de l'infarctus, les porcs peuvent subir une administration endocardique percutanée du biomatériau imprégné de VE ou du biomatériau sans VE pour les témoins. Après anesthésie, un fil de guidage peut être avancé dans le ventricule gauche à travers la valve aortique précédant l'insertion d'un cathéter à queue de cochon sur le fil de guidage. La ventriculographie gauche peut alors être effectuée à la fois dans les vues antéro-postérieures et latérales pour délimiter les régions de dysfonctionnement myocardique. Un cathéter dédié (C-Cath®, Celyad, Mont-Saint-Guibert, Belgique) peut être inséré à travers une gaine et avancé vers le ventricule gauche sous fluoroscopie. le cathéter dispose d'une aiguille courbe de 75° avec des trous latéraux gradués de petite à grande taille. Les gels contenant les VE peuvent être injectés à une vitesse d'environ 0,5 ml / min dans environ 10 sites, en attendant quelques secondes avant le retrait de l'aiguille pour minimiser les fuites rétrogrades.

Les analyses de l'effet des administration sont réalisée 3 mois après l'injection. Le critère principal est le résultat fonctionnel évalué par échocardiographie et imagerie par résonance magnétique (IRM) effectuée immédiatement avant le traitement (c.-à-d., 3 semaines après l'infarctus) et au moment du sacrifice. Les séquences d'IRM seront définies pour évaluer la fonction LV (volumes et fraction d'éjection), la perfusion myocardique et la taille de l'infarctus après l'injection de gadolinium. Après le sacrifice, les coeurs explantés sont traités pour des évaluations histologiques standard (taille de l'infarctus) et des analyses immunohistochimiques (angiogenèse, infiltration de cellules inflammatoires, polarisation des macrophages). De plus, des échantillons de sang sont prélevés avant le traitement et au moment du sacrifice pour l'analyse du peptide atrialnatriurétique. Les fragments du myocarde subissent également une analyse transcriptomique pour rechercher l'expression différentielle des gènes impliqués dans les voies qui contribuent à la préservation du myocarde (survie et prolifération des cellules, angiogenèse et vasculogenèse). Enfin, des biopsies de divers organes (poumons, foie, reins) sont effectuées pour vérifier l'absence de prolifération anormale des tissus. Toutes les données seront recueillies et analysées en aveugle.

L'approche implant sous forme de gel / patch est justifiée pour deux raisons. Tout d'abord, certains patients ont besoin d'une intervention chirurgicale cardiaque après avoir subi un infarctus du myocarde et peuvent bénéficier d'une réparation myocardique grâce au matériau de l'invention imprégné de VE, déposé sous la forme d'un implant et/ou d'un gel sur l'épicarde de la zone nécrosée. Ainsi, une autre série d'expériences, ciblées sur ces applications chirurgicales, peut être effectuée sur les porcs après création d'un infarctus selon la procédure interventionnelle décrite plus haut. Le coeur est alors abordé par thoracotomie gauche 3 semaines après la création de l'infarctus et après repérage visuel de la zone infarcie, le matériau de l'invention imprégné de VE est déposé sous la forme d'un implant et/ou d'un gel sur l'épicarde de cette zone en débordant ses limites. La thoracotomie est ensuite refermée. Trois mois plus tard, les animaux sont euthanasiés pour évaluation selon des critères identiques à ceux décrits plus haut à propos des traitements effectués par voie percutanée endoventriculaire gauche.

**Références bibliographiques**

**[1]** Pr. Philippe Menasché et al., - Towards a clinical use of human embryonic stem cell-derived cardiac progenitors : a translational expérience - European Society of Cardiology, European Heart Journal (2015) 36, 743-750.

[2] Anaïs Kervadec et al. - Cardiovascular progenitor-derived extracellular vesicle recapitulate the bénéficiai effect of their parent cells in threatment of chronic heart failure - Elsevier, Original Pre-clinic Science, The Journal of Heart and Lung Transplantation, January 13, 2016, 35:795-807.

[3] US 5,750,657 - Bristol-Myers Squibb Company.

[4] US 5,770,194 - Bristol-Myers Squibb Company.

[5] US 5,773,418 - Bristol-Myers Squibb Company.

[6] US 5,804,428 - Bristol-Myers Squibb Company.

[7] Wong et al., « Fibrin-based biomaterials to deliver human growth factors », Thromb Haemost 2003;89:573-82

[8] Spicer et al. Fibrin glue as a drug delivery system. J Control Release 2010;148:49-55

[9] Whelan et al., « Fibrin as a delivery system in wound healing tissue engineering applications », J Control Release 2014;196:1-8

[10] Ahmad et al. Fibrin matrices: « The versatile therapeutic delivery systems », International Journal of Biomolecules 2015;81:121-36.

[11] Bellamy et al., « Long-term functional benefits of human embryonic stem cell-derived cardiac progenitors embedded into a fibrin scaffold », J Heart Lung Transplant. 2015 Sep;34 :1198-207.

[12] Pr. Philippe Menasché et al., « Towards a clinical use of human embryonic stem cell-derived cardiac progenitors: a translational expérience », Eur Heart J. 2015;36:743-50.

[13] T. Chandy et CP Sharma chitosane comme biomatériau Biomat, Art. Cells, Art. Org, 18; 1-24 1990.

**[14]** Ladage et al., « Stimulating myocardial régénération with periostin Peptide in large mammals improves fonction post-myocardial infarction but increases myocardial fibrosis », PLoS One. 2013;8:e59656.

**[15]** Polizzoti et al. « Intrapericardial delivery of gelfoam enables the targeted delivery of Periostin peptide after myocardial infarction by inducing fibrin clot formation », PLoS One, 2012 ;7 :e36788.

**[16]** Morcuende et al., « Neuroprotective effects of NGF, BDNF, NT-3 and GDNF on axotomized extraocular motoneurons in neonatal rats », Neuroscience. 2013;250:31-48.

**[17]** Wei et al., « Epicardial FSTL1 reconstitution regenerates the adult mammalian heart », Nature. 2015 Sep 24;525(7570):479-85.

**[18]** Jo et al., « Sequential delivery of BMP-2 and BMP-7 for bone régénération using a heparinized collagen membrane », Int J Oral Maxillofac Surg. 2015;44:921-8.).

**[19]** Oliver R. et al, 1976, Clin.. Orthop 115: 291-30; 1980, Rr J. Exp Chemin 61, 544-549; 1981, Conn Tissue Res 9: 59-62.

**[20]** Skjak-Braerk, G.; Grasdalen, H.; Smidsrod, O. Inhomogeneous polysaccharide ionic gels. Carbohydr. Polym. 1989, 10, 31-54.

**[21]** Kolambkar et al., « An alginate-based hybrid system for growth factor delivery in the functional repair of large bone defects », Biomaterials 2011 ;32 :65-74.

**[22]** Ruvinov et al., « The effects of controlled HGF delivery from an affinity-binding alginate biomaterial on angiogenesis and blood perfusion in a hindlimb ischemia model », Biomaterials 2010 ; 31 :4573-4582.

**[23]** Mann et al., « One-year follow-up results from AUGMENT-HF: a multicentre randomized controlled clinical trial of the efficacy of left ventricular augmentation with Algisyl in the treatment of heart failure », Eur J Heart Fail. 2016;18:314-25.

**[24]** Silva et al., « Delivery of LLKKK18 loaded into self-assembling hyaluronic acid nanogel for tuberculosis treatment », J Control Release. 2016 Aug 10;235:112-24.

**[25]** Soranno et al., « Delivery of interleukin-10 via injectable hydrogels improves renal outcomes and reduces systemic inflammation following ischémie acute kidney injury in mice » Am J Physiol Renal Physiol. 2016;311:F362-72.

**[26]** Eckhouse et al., « Local hydrogel release of recombinant TIMP-3 atténuâtes adverse left ventricular remodeling after experimental myocardial infarction », Sci Transl Med. 2014;6:223ra21.

**[27]** Landi et al., « Hyaluronic acid scaffold for skin defects in congenital syndactyly release surgery: a novel technique based on the regenerative model », J Hand Surg Eur Vol. 2014 Nov;39(9):994-1000.

**[28]** Gutowski KA., « Hyaluronic acid fillers: Science and clinical uses », Clin Plast Surg. 2016;43:489-96.

**[29]** WO2007025233 - Regents Of The University Of Minnesota.

**[30]** Khorramirouz R et al., « Effect of three decellularisation protocols on the mechanical behaviour and structural properties of sheep aortic valve conduits », Adv Med Sci. 2014;59:299-307.

**[31]** He. et al., « Optimizationof SDS exposure on préservation of ECM characteristics in whole organ decellularization of rat kidneys », J Biomed Mater Res B Appl Biomater. 2016 Apr 8. doi: 10.1002/jbm.b.33668.

**[32]** Seif-Naraghi et al. « Safety and efficacy of an injectable extracellular matrix hydrogel for treating myocardial infarction », Sci Transl Med 2013 ;5:173ra25.

**[33]** Mohiti-Asli et al; Ibuprofen loaded PLA nanofibrous scaffolds increase prolifération of human skin cells in vitro and promote healing of full thickness incision wounds in vivo. J Biomed Mater Res B Appl Biomater. 2015 Oct 28. doi: 10.1002/jbm.b.33520.

**[34]** Tyler et al., « Polylactic acid (PLA) controlled delivery carriers for biomédical applications », Adv Drug Deliv Rev. 2016 Jul 15. pii: S0169-409X(16)30211-3. doi: 10.1016/j.addr.2016.06.018.

**[35]** James et al., « Poly(lactic acid) for delivery of bioactive macromolecules » Adv Drug Deliv Rev. 2016 Jun 25. pii: S0169-409X(16)30193-4. doi: 10.1016/j.addr.2016.06.009.

[36] Liu et al. « HB-EGF embedded in PGA/PLLA scaffolds via subcritical CO2 augments the production of tissue engineered intestine. Biomaterials », 2016;103:150-9.

[37] Thatcher et al ; « Thymosin β4 sustained release from poly(lactide-co-glycolide) microspheres: synthesis and implications for treatment of myocardial ischemia », Ann N Y Acad Sci. 2012;1270:112-9.

[38] Arias JA et al., « Synthesis, degradability, and drug releasing properties of methyl esters of fungal poly(beta,L-malic acid) », Macromol Biosci. 2008;8:540-50.

[39] Loyer et al., « Natural and synthetic poly(malic acid)-based dérivâtes: a family of versatile biopolymers for the design of drug nanocarriers » J Drug Target. 2014;22:556-75.

[40] Patel JJ, et al., « Dual delivery of EPO and BMP2 from a novel modular poly-ε-caprolactone construct to increase the bone formation in prefabricated bone flaps », Tissue Eng Part C Methods. 2015;21:889-97.

[41] Singh S, et al. The enhancement of VEGF-mediated angiogenesis by polycaprolactone scaffolds with surface cross-linked heparin. Biomaterials 2011 ;32 :2059-2069.

[42] Jeong et al., « Three-dimensional polycaprolactone scaffold-conjugated bone morphogenetic protein-2 promotes cartilage régénération from primary chondrocytes in vitro and in vivo without accelerated endochondral ossification », J Biomed Mater Res A. 2012;100:2088-96.

[43] Takahashi et Yamanaka, « A decade of transcription factor-mediated reprogramming to pluripotency. », Nat Rev Mol Cell Biol. 2016;17:183-93.

**[44]** Barile et al., « Extracellular vesicles from human cardiac progenitor cells inhibit cardiomyocyte apoptosis and improve cardiac function aftermyocardial infarction », Cardiovasc Res 2014;103:530-41.

**[45]** Bobis-Wozowicz et al., « Human induced pluripotent stem cell-derived microvesicles transmit RNAs and proteins to récipient mature heart cells modulating cell fate and behavior », Stem Cells. 2015;33:2748-61.

**[46]** Lai et al. Exosome secreted by MSC reduces myocardial ischemia/reperfusion injury. Stem cell Res 2012;4:214-22.

**[47]** Baulch et al. Cranial grafting of stem cell-derived microvesicles improves cognition and reduces neuropathology in the irradiated brain. Proc Natl Acad Sci USA. 2016;113:4836-41.

**[48]** Nojima H et. al - Hepatocyte exosomes médiate liver repair and régénération via sphingosine-1-phosphate - J Hepatol. 2016 Jan;64(1):60-8.

**[49]** Herrera MB et. al - Human liver stem cell-derived microvesicles accelerate hepatic régénération in hepatectomized rats - J Celle Mol Med. 2010 Jun;14(GB):1605-18.

**[50]** Besse B et. al - Dendritic cell-derived exosomes as maintenance immunotherapy after first line chemotherapy in NSCLC - Oncoimmunology. 2015 Aug 12;5(4):e1071008. eCollection 2016.

**[51]** Alessandra Fierabracci et. al - Recent Advances in Mesenchymal Stem Cell Immunomodulation: The Role of Microvesicles - Cell Transplantation, Vol. 24, pp. 133-149, 2015.

**[52]** Mirjam C. Boelens et. al - Exosome Transfer from Stromal to Breast Cancer Cells Regulates Therapy Résistance Pathways - EMD Millipore Volume 159, Issue 3, p499-513, 23 October 2014 Amnis Imaging Flow Cytometry Integrating flow cytometry and microscopy.

**[53]** Paul D. Robbins et. al - Regulation of Immune Responses by Extracellular Vesicles - Nat Rev Immunol. 2014 March ; 14(3): 195-208.

**[54]** Bin Zhang et. al - Mesenchymal Stem Cells Secrete Immunologically Active Exosomes - Stem Cells and Development, Volume 23, Number 11, 2014 Mary Ann Liebert, Inc..

[55] L Kordelas et. al - MSC-derived exosomes: a novel tool to treat therapy-refractory graft-versus-host disease - Leukemia 2014;28;970 -3.

[56] Budoni M et. al - The immunosuppressive effect of mesenchymal stromal cells on B lymphocytes is mediated by membrane vesicles - Cell Transplant. 2013;22(2):369-79.

[57] Christianson HC et. al - Cancer cell exosomes dépend on cell-surface heparin sulfate proteoglycans for their internalization and functional activity - Proc Natl Acad Sci USA. 2013 Oct 22;110(43):17380-5.

[58] Ratajczak J et. al - Embryonic stem cell-derived microvesicles reprogram hematopoietic progenitors: évidence for horizontal transfer of mRNA and protein delivery - Leukemia. 2006 May;20(5):847-56.

[59] Morelli AE et. al - The immune regulatory effect of apoptotic cells and exosomes on dendritic cells: its impact on transplantation - Am J Transplant. 2006 Feb;6(2):254-61.

[60] Pêche H et. al - Présentation of donor major histocompatibility complex antigens by bone marrow dendritic cell-derived exosomes modulates allograft rejection - Transplantation. 2003 Nov 27;76(10):1503-10.

[61] Celine Akyurekli et. al - A Systematic Review of Preclinical Studies on the Therapeutic Potential of Mesenchymal Stromal Cell-Derived Microvesicles - Stem Cell Rev and Rep, DOI 10.1007/s12015-014-9545-9, 05 August 2014.

[62] Sarah E et. al - Neutrophil-derived microvesicles enter cartilage and protect the joint in inflammatory arthritis - Science Translational Medicine 25 November 2015 Vol 7 Issue 315 315ra190.

[63] Bin Zhang et. al - HucMSC-exosome Mediated - Wnt4 Signaling is Required for Cutaneous Wound Healing - Stem Cells 2014;00:00-00 AlphaMed Press 2014.

**[64]** Rekha Nair et. al - Extracellular Vesicles Derived from Preosteoblasts Influence Embryonic Stem Cell Différentiation - Stem Cells and Development Volume 23, Number 14, 2014 Mary Ann Liebert, Inc..

**[65]** Vincenzo Cantaluppi et. al - Microvesicles Derived From Endothelial Progenitor Cells Enhance Neoangiogenesis of Human Pancreatic Islets - Cell Transplantation. Vol. 21. Pp. 1305-1320. 2012.

**[66]** Hae Kyung et. al - Mesenchymal stem cells deliver exogenous miRNAs to neural cells and induce their différentiation and glutamate transporter expression - Delivery of miRNAs by MSCs to neural cells. Stem Cells Dev. 2014; 23:2851-61.

**[67]** Hongqi Xin et. al - Systemic administration of exosomes released from mesenchymal stromal cells promote functional recovery and neurovascular plasticity after stroke in rats - Journal of Cerebral Blood Flow & Metabolism (2013), 1-5 2013 ISCBFM.

**[68]** Jian-ying Chen et. al - Therapeutic effects of mesenchymal stem cell-derived microvesicles on pulmonary arterial hypertension in rats-Acta Pharmacologica Sinica, 4 August 2014.

**[69]** Lee C et. al - Exosomes médiate the cytoprotective action of mesenchymal stromal cells on hypoxia-induced pulmonary hypertension - Circulation. 2012 Nov 27;126(22):2601-11.

**[70]** Islam MN et al. - Mitochondrial transfer from bone-marrow-derived stromal cells to pulmonary alveoli protects against acute lung injury - Mitochondria to the rescue, Nat Med. 2012.

**[71]** Changjin Lee et. al - Exosomes Médiate the Cytoprotective Action of Mesenchymal Stromal Cells on Hypoxia-Induced Pulmonary Hypertension - Circulationaha.112.114173 Published online before print October 31, 2012.

**[72]** Aliotta JM et. al - Stable cell fate changes in marrow cells induced by lung-derived microvesicles - J Extracell Vesicles. 2012 Apr 16;1.

**[73]** Der-Cherng Targ et. al - Induced Pluripotent Stem Cell-Derived Conditioned Medium Attenuates Acute Kidney Injury by Downregulating the Oxidative Stress-Related Pathway in Ischemia-Reperfusion Rats - Cell Transplantation, Vol. 25, pp. 517-530, 2016.

[74] Bruno S et. al - Microvesicles derived from mesenchymal stem cells enhance survival in a lethal model of acute kidney injury - PloS One. 2012;7(3):e33115.

[75] Cristina Grange et. al - Biodistribution of mesenchymal stem cell-derived extracellular vesicles in a model of acute kidney injury monitored by optical imaging - International Journal of Molecular Medicine 33: 1055-1063, 2014.

[76] Grange C et. al - Biodistribution of mesenchymal stem cell-derived extracellular vesicles in a model of acute kidney injury monitored by optical imaging - Int J Mol Med. 2014 May;33(5):1055-63.

[77] Cantaluppi V et. al - Microvesicles derived from endothelial progenitor cells protect the kidney from ischemia-reperfusion injury by microRNA-dependent reprogramming of résident renal cells - Kidney Int. 2012 Aug;82(4):412-27.

[78] Vincenzo Cantaluppi et. al - Microvesicles derived from endothelial progenitor cells protect the kidney from ischemia-reperfusion injury by microRNA-dependent reprogramming of résident renal cells - Kidney International advance online publication 11 April 2012.

[79] Joseph V. Bonventre et. al - Microvesicles from Mesenchymal Stromal Cells Protect against Acute Kidney Injury - J Am Soc Nephrol 20: 925-932, 2009.

[80] Stefania Bruno et. al - Mesenchymal Stem Cell-Derived Microvesicles Protect Against Acute Tubular Injury - J Am Soc Nephrol 20: 1053-1067, 2009.

[81] Janet E. et. al - Cranial grafting of stem cell-derived microvesicles improves cognition and reduces neuropathology in the irradiated brain - PNAS Early Edition - www.pnas.og/cgi/doi/10.1073/pnas.1521668113.

**[82]** Masako Nakano et. al - Bone marrow-derived mesenchymal stem cells improve diabetes-induced cognitive impairment by exosome transfer into damaged neurons and astrocytes - Scientific reports/6:24805/.

[83] Krishnan et al. « Engineering a growth factor embedded nanofiber matrix niche to promote vascularization for functional cardiac régénération » Biomaterials. 2016;97:176-95.

[84] Lu Y, et al., « Coaxial electrospun fibers: applications in drug delivery and tissue engineering », Wiley Interdiscip Rev Nanomed Nanobiotechnol. 2016 Feb 5. doi: 10.1002/wnan.1391. [Epub ahead of print].

[85] Gaetani R, et al., « Epicardial application of cardiac progenitor cells in a 3D-printed gelatin/hyaluronic acid patch préserves cardiac function after myocardial infarction », Biomaterials. 2015;61:339-48.

[86] Gray WD et al, Identification of therapeutic covariant microRNA clusters in hypoxia-treated cardiac progenitor cell exosomes using systems biology. Circ. Res. 2015 Jan16 ; 116(2) :255-63.

## Revendications

1. Biomatériau comprenant un polymère biocompatible biodégradable incluant des vésicules extracellulaires de cellules souches pluripotentes ou multipotentes ou de cellules progénitrices cardiaques, lesdites vésicules étant un ou plusieurs éléments produits par lesdites cellules choisis parmi les microparticules, les exosomes et les corps apoptotiques, dans lequel ledit polymère biocompatible biodégradable est un polymère de fibrine ayant une taille des pores comprise entre 50 $\mu$m et 200 $\mu$m, une épaisseur comprise entre 0,8 mm et 1,2 mm et une élasticité comprise entre 6 kPa et 15 kPa.

2. Biomatériau selon la revendication 1, dans lequel le biomatériau est obtenu par un procédé comprenant une étape de trempage du polymère biocompatible biodégradable dans un milieu liquide biocompatible comprenant des vésicules extracellulaires de cellules souches pluripotentes ou multipotentes ou de cellules progénitrices cardiaques ou par mélange du polymère biocompatible biodégradable et/ou du ou des monomères correspondants avec des vésicules extracellulaires de cellules souches ou progénitrices cardiaques.

3. Procédé de fabrication d'un biomatériau tel que défini dans l'une quelconque des revendications 1 à 2 comprenant une étape de re-suspension des vésicules extracellulaires de cellules souches pluripotentes ou multipotentes ou de cellules progénitrices cardiaques dans une solution aqueuse de fibrinogène, puis une étape de mélange de cette solution avec une solution de thrombine, lesdites vésicules étant un ou plusieurs éléments produits par lesdites cellules choisis parmi les microvésicules, les exosomes et les corps apoptotiques.

## Patentansprüche

1. Biomaterial, das ein biologisch abbaubares, biokompatibles Polymer umfasst, das extrazelluläre Vesikel pluripotenter oder multipotenter Stammzellen oder kardialer Vorläuferzellen einschließt, wobei die Vesikel ein oder mehrere Elemente sind, die von den Zellen erzeugt werden, ausgewählt aus Mikropartikeln, Exosomen und apoptotischen Körpern, wobei das biologisch abbaubare, biokompatible Polymer ein Fibrinpolymer mit einer Porengröße zwischen 50 $\mu$m und 200 $\mu$m, einer Dicke zwischen 0.8 mm und 1,2 mm und einer Elastizität zwischen 6 kPa und 15 kPa.

2. Biomaterial nach Anspruch 1, wobei das Biomaterial durch ein Verfahren erhalten wird, das einen Schritt des Einweichens des bioabbaubaren biokompatiblen Polymers in einem biokompatiblen flüssigen Medium, das extrazelluläre Vesikel pluripotenter oder multipotenter Stammzellen oder kardialer Vorläuferzellen umfasst, oder durch Mischen des bioabbaubaren biokompatiblen Polymers und/oder des/der entsprechenden Monomers/Monomer mit extrazellulären Vesikeln von Stammzellen oder kardialen Vorläuferzellen umfasst.

3. Verfahren zur Herstellung eines Biomaterials nach einem der Ansprüche 1 bis 2, umfassend einen Schritt des Resuspendierens extrazellulärer Vesikel pluripotenter oder multipotenter Stammzellen oder kardialer Vorläuferzellen in einer wässrigen Fibrinogenlösung, gefolgt von einem Schritt des Mischens dieser Lösung mit einer Thrombinlösung, wobei die Vesikel ein oder mehrere Elemente sind, die von den Zellen produziert werden, ausgewählt aus Mikrovesikeln, Exosomen und apoptotischen Körpern.

## Claims

1. A biomaterial comprising a biodegradable biocompatible polymer including extracellular vesicles of pluripotent or multipotent stem cells or cardiac progenitor cells, said vesicles being one or more elements produced by said cells selected from microparticles, exosomes and apoptotic bodies, wherein said biocompatible biodegradable polymer is a fibrin polymer having a pore size of between 50 $\mu$m and 200 $\mu$m, a thickness of between 0.8 mm and 1.2 mm and an elasticity of between 6 kPa and 15 kPa.

2. The biomaterial of claim 1, wherein the biomaterial is obtained by a process comprising a step of soaking the biodegradable biocompatible polymer in a biocompatible liquid medium comprising extracellular vesicles of pluripotent or multipotent stem cells or cardiac progenitor cells or by mixing the biodegradable biocompatible polymer and/or the corresponding monomer(s) with extracellular vesicles of stem cells or cardiac progenitor cells.

3. A method of manufacturing a biomaterial as defined in any one of claims 1 to 2 comprising a step of resuspending extracellular vesicles of pluripotent or multipotent stem cells or cardiac progenitor cells in an aqueous fibrinogen solution, followed by a step of mixing this solution with a thrombin solution, said vesicles being one or more elements produced by said cells selected from microvesicles, exosomes and apoptotic bodies.

a
b
c
d
e
f
200 nm
200 nm
200 nm
200 nm
200 nm
200 nm

**Figure 1**

| Réplicat | Concentration | Particules/ cellule | Mode (nm) | particules 50-150 nm | particules 151-500 nm | particules 501-1000 nm |
|---|---|---|---|---|---|---|
| 1 | 6,2 X $10^8$ p/uL | 16 702 | 97,2 | 63,4% | 34,1% | 2,2% |
| 2 | 4,2 X $10^8$ p/uL | 13 650 | 86,6 | 77,9% | 20% | 1,3% |
| 3 | 6,0 X $10^8$ p/uL | 16 378 | 97,4 | 64,2% | 33,8% | 1,6% |

**Figure 2**

iPS-Pg
iPS-Pg-EV
CD63
CD81
B-ACTIN

**Figure 3**

A
B
C
D

**Figure 4**

A
B
C
D
E

Figure 5

Elasticité (kPa, ± SEM)
70
60
50
40
30
20
10
0
N/A
20,93
4,27
4,055
59,06
10,44
F10T2
F15T3
F20T4
F25T5
F30T6
F35T7

Figure 6

F5T1

F5T1+VE

F20T4

F20T4+VE

J0, 40X

J0, 63X

J2, 40X

J2, 63X

Figure 7

Calcéine
Microscopie optique
10 µm
10 µm
F5T1
F20T4

Figure 8

a
100
75
50
25
0
% de cicatrisation
Contrôle positif
Contrôle négatif
1.00E+07
3.00E+08
1.00E+09
3.00E+09
6.00E+09
1.00E+10
Nombre de VE-iPS-Pg

b
c
d
Contrôle positif
(milieu complet)
Contrôle négatif
(milieu pauvre)
Milieu pauvre
+ VE-iPS-Pg

**Figure 9**

200
150
100
50
0
% de cellules viables à 27h
T0
Contrôle positif
Contrôle négatif
EV dérivées de FBS
VE-iPs-Pg
6.6E+06
66E+06
662E+06
2.65E+09
5.3E+09
13.2E+09
53E+09
80E+09
Nombre de vésicules extracellulaires

**Figure 10**

a

Fréquence normalisée
Intensité de fluorescence
0
0.5
1
1.5
2
2.5
3
0
1e3
1e4
1e5
1e6
1e7
1
2,3
4
5
6
7
8


b

Lumière blanche
Calcein AM
Lumière blanche
Calcein AM
1
2
3
4
5
6
7
8


Figure 11

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2013169202 A
- WO 2013142878 A
- US 5750657 A
- US 5770194 A
- US 5773418 A
- US 5804428 A
- WO 2007025233 A


### Littérature non-brevet citée dans la description


- **PR. PHILIPPE MENASCHÉ et al.** Towards a clinical use of human embryonic stem cell-derived cardiac progenitors : a translational expérience. *European Heart Journal,* 2015, vol. 36, 743-750
- Cardiovascular progenitor-derived extracellular vesicles recapitulate the bénéficiai effect of their parent cells in the treatment of chronic heart failure. **ANAÏS KERVADEC ; PR. PHILIPPE MENASCHÉ et al.** Original Pre-clinic Science, The Journal of Heart and Lung Transplantation. Elsevier, 13 Janvier 2016, vol. 35, 795-807
- **WONG et al.** Fibrin-based biomaterials to deliver human growth factors. *Thromb Haemost,* 2003, vol. 89, 573-82
- **SPICER et al.** *Fibrin glue as a drug delivery system. J Control Release,* 2010, vol. 148, 49-55
- **WHELAN et al.** Fibrin as a delivery system in wound healing tissue engineering applications. *J Control Release,* 2014, vol. 196, 1-8
- **AHMAD et al.** The versatile therapeutic delivery systems. *International Journal of Biomolecules,* 2015, vol. 81, 121-36
- **BELLAMY et al.** Long-term functional benefits of human embryonic stem cell-derived cardiac progenitors embedded into a fibrin scaffold. *J Heart Lung Transplant.,* Septembre 2015, vol. 34, 1198-207
- **PR. PHILIPPE MENASCHÉ et al.** Towards a clinical use of human embryonic stem cell-derived cardiac progenitors: a translational expérience. *Eur Heart J.,* 2015, vol. 36, 743-50
- **T. CHANDY ; CP SHARMA.** chitosane comme biomatériau. *Biomat, Art. Cells, Art. Org,* 1990, vol. 18, 1-24
- **LADAGE et al.** Stimulating myocardial régénération with periostin Peptide in large mammals improves function post-myocardial infarction but increases myocardial fibrosis. *PLoS One.,* 2013, vol. 8, e59656
- **POLIZZOTI et al.** Intrapericardial delivery of gelfoam enables the targeted delivery of Periostin peptide after myocardial infarction by inducing fibrin clot formation. *PLoS One,* 2012, vol. 7, e36788
- **MORCUENDE et al.** Neuroprotective effects of NGF, BDNF, NT-3 and GDNF on axotomized extraocular motoneurons in neonatal rats. *Neuroscience.,* 2013, vol. 250, 31-48
- **WEI et al.** Epicardial FSTL1 reconstitution regenerates the adult mammalian heart. *Nature,* 24 Septembre 2015, vol. 525 (7570), 479-85
- **JO et al.** Sequential delivery of BMP-2 and BMP-7 for bone régénération using a heparinized collagen membrane. *Int J Oral Maxillofac Surg.,* 2015, vol. 44, 921-8
- **OLIVER R. et al.** *Clin.. Orthop,* 1976, vol. 115, 291-30
- *Rr J. Exp Chemin,* 1981, vol. 61, 544-549
- *Conn Tissue Res,* vol. 9, 59-62
- **SKJAK-BRAERK, G ; GRASDALEN, H ; SMIDSROD, O.** Inhomogeneous polysaccharide ionic gels. *Carbohydr. Polym.,* 1989, vol. 10, 31-54
- **KOLAMBKAR et al.** An alginate-based hybrid system for growth factor delivery in the functional repair of large bone defects. *Biomaterials,* 2011, vol. 32, 65-74
- **RUVINOV et al.** The effects of controlled HGF delivery from an affinity-binding alginate biomaterial on angiogenesis and blood perfusion in a hindlimb ischemia model. *Biomaterials,* 2010, vol. 31, 4573-4582
- **MANN et al.** One-year follow-up results from AUGMENT-HF: a multicentre randomized controlled clinicaltrial of the efficacy of left ventricular augmentation with Algisyl in the treatment of heart failure. *Eur J Heart Fail.,* 2016, vol. 18, 314-25
- **SILVA et al.** Delivery of LLKKK18 loaded into self-assembling hyaluronic acid nanogel for tuberculosis treatment. *J Control Release.,* 10 Août 2016, vol. 235, 112-24

- **SORANNO et al.** Delivery of interleukin-10 via injectable hydrogels improves renal outcomes and reduces systemic inflammation following ischémie acute kidney injury in mice. *Am J Physiol Renal Physiol.,* 2016, vol. 311, F362-72
- **ECKHOUSE et al.** Local hydrogel release of recombinant TIMP-3 atténuâtes adverse left ventricular remodeling after experimental myocardial infarction. *Sci Transl Med.,* 2014, vol. 6, 223-21
- **LANDI et al.** Hyaluronic acid scaffold for skin defects in congenital syndactyly release surgery: a novel technique based on the regenerative model. *J Hand Surg Eur,* Novembre 2014, vol. 39 (9), 994-1000
- **GUTOWSKI KA.** Hyaluronic acid fillers: Science and clinical uses. *Clin Plast Surg.,* 2016, vol. 43, 489-96
- **KHORRAMIROUZ R et al.** Effect of three decellularisation protocols on the mechanical behaviour and structural properties of sheep aortic valve conduits. *Adv Med Sci.,* 2014, vol. 59, 299-307
- **HE et al.** Optimizationof SDS exposure on préservation of ECM characteristics in whole organ decellularization of rat kidneys. *J Biomed Mater Res B Appl Biomater.,* 08 Avril 2016
- **SEIF-NARAGHI et al.** Safety and efficacy of an injectable extracellular matrix hydrogel for treating myocardial infarction. *Sci Transl Med,* 2013, vol. 5, 173-25
- **MOHITI-ASLI et al.** Ibuprofen loaded PLA nanofibrous scaffolds increase prolifération of human skin cells in vitro and promote healing of full thickness incision wounds in vivo. *J Biomed Mater Res B Appl Biomater.,* 28 Octobre 2015
- **TYLER et al.** Polylactic acid (PLA) controlled delivery carriers for biomedical applications. *Adv Drug Deliv Rev.,* 15 Juillet 2016, S0169-409
- **JAMES et al.** Poly(lactic acid) for delivery of bioactive macromolecules. *Adv Drug Deliv Rev,* 25 Juin 2016, S0169-409
- **LIU et al.** HB-EGF embedded in PGA/PLLA scaffolds via subcritical CO2 augments the production of tissue engineered intestine. *Biomaterials,* 2016, vol. 103, 150-9
- **THATCHER et al.** Thymosin β4 sustained release from poly(lactide-co-glycolide) microspheres: synthesis and implications for treatment of myocardial ischemia. *Ann N Y Acad Sci.,* 2012, vol. 1270, 112-9
- **PORTILLA-ARIAS JA et al.** Synthesis, degradability, and drug releasing properties of methyl esters of fungal poly(beta,L-malic acid). *Macromol Biosci.,* 2008, vol. 8, 540-50
- **LOYER et al.** Natural and synthetic poly(malic acid)-based dérivâtes: a family of versatile biopolymers for the design of drug nanocarriers. *J Drug Target.,* 2014, vol. 22, 556-75
- **PATEL JJ et al.** Dual delivery of EPO and BMP2 from a novel modular poly-ε-caprolactone construct to increase the bone formation in prefabricated bone flaps. *Tissue Eng Part C Methods.,* 2015, vol. 21, 889-97
- **SINGH S et al.** The enhancement of VEGF-mediated angiogenesis by polycaprolactone scaffolds with surface cross-linked heparin. *Biomaterials,* 2011, vol. 32, 2059-2069
- **JEONG et al.** Three-dimensional polycaprolactone scaffold-conjugated bone morphogenetic protein-2 promotes cartilage régénération from primary chondrocytes in vitro and in vivo without accelerated endochondral ossification. *J Biomed Mater Res A.,* 2012, vol. 100, 2088-96
- **TAKAHASHI ; YAMANAKA.** A decade of transcription factor-mediated reprogramming to pluripotency. *Nat Rev Mol Cell Biol.,* 2016, vol. 17, 183-93
- **BARILE et al.** Extracellular vesicles from human cardiac progenitor cells inhibit cardiomyocyte apoptosis and improve cardiac function aftermyocardial infarction. *Cardiovasc Res,* 2014, vol. 103, 530-41
- **BOBIS-WOZOWICZ et al.** Human induced pluripotent stem cell-derived microvesicles transmit RNAs and proteins to récipient mature heart cells modulating cell fate and behavior. *Stem Cells.,* 2015, vol. 33, 2748-61
- **LAI et al.** Exosome secreted by MSC reduces myocardial ischemia/reperfusion injury. *Stem cell Res,* 2012, vol. 4, 214-22
- **BAULCH et al.** Cranial grafting of stem cell-derived microvesicles improves cognition and reduces neuropathology in the irradiated brain. *Proc Natl Acad Sci USA.,* 2016, vol. 113, 4836-41
- **NOJIMA H.** Hepatocyte exosomes médiate liver repair and régénération via sphingosine-1-phosphate. *J Hepatol.,* Janvier 2016, vol. 64 (1), 60-8
- **HERRERA MB.** Human liver stem cell-derived microvesicles accelerate hepatic régénération in hepatectomized rats. *J Celle Mol Med.,* Juin 2010, vol. 14, 1605-18
- **BESSE B.** Dendritic cell-derived exosomes as maintenance immunotherapy after first line chemotherapy in NSCLC. *Oncoimmunology,* 12 Août 2015, vol. 5 (4), e1071008
- **ALESSANDRA FIERABRACCI.** Recent Advances in Mesenchymal Stem Cell Immunomodulation: The Role of Microvesicles. *Cell Transplantation,* 2015, vol. 24, 133-149
- **MIRJAM C. BOELENS.** Exosome Transfer from Stromal to Breast Cancer Cells Regulates Therapy Résistance Pathways. *EMD Millipore,* 23 Octobre 2014, vol. 159 (3), 499-513

- **PAUL D. ROBBINS.** Regulation of Immune Responses by Extracellular Vesicles. *Nat Rev Immunol.,* Mars 2014, vol. 14 (3), 195-208
- **BIN ZHANG.** Mesenchymal Stem Cells Secrete Immunologically Active Exosomes. *Stem Cells and Development,* 2014, vol. 23 (11
- **L KORDELAS.** MSC-derived exosomes: a novel tool to treat therapy-refractory graft-versus-host disease. *Leukemia,* 2014, vol. 28, 970-3
- **BUDONI M.** The immunosuppressive effect of mesenchymal stromal cells on B lymphocytes is mediated by membrane vesicles. *Cell Transplant.,* 2013, vol. 22 (2), 369-79
- **CHRISTIANSON HC.** Cancer cell exosomes dépend on cell-surface heparin sulfate proteoglycans for their internalization and functional activity. *Proc Natl Acad Sci USA.,* 22 Octobre 2013, vol. 110 (43), 17380-5
- **RATAJCZAK J.** Embryonic stem cell-derived microvesicles reprogram hematopoietic progenitors: évidence for horizontal transfer of mRNA and protein delivery. *Leukemia.,* Mai 2006, vol. 20 (5), 847-56
- **MORELLI AE.** The immune regulatory effect of apoptotic cells and exosomes on dendritic cells: its impact on transplantation. *Am J Transplant.,* Février 2006, vol. 6 (2), 254-61
- **PÊCHE H.** Présentation of donor major histocompatibility complex antigens by bone marrow dendritic cell-derived exosomes modulates allograft rejection. *Transplantation.,* 27 Novembre 2003, vol. 76 (10), 1503-10
- **CELINE AKYUREKLI.** A Systematic Review of Preclinical Studies on the Therapeutic Potential of Mesenchymal Stromal Cell-Derived Microvesicles. *Stem Cell Rev and Rep,* 05 Août 2014, 9545-9
- **SARAH E.** Neutrophil-derived microvesicles enter cartilage and protect the joint in inflammatory arthritis. *Science Translational Medicine,* 25 Novembre 2015, vol. 7 (315), 315-190
- **BIN ZHANG.** HucMSC-exosome Mediated - Wnt4 Signaling is Required for Cutaneous Wound Healing. *Stem Cells,* 2014, vol. 00, 00-00
- **REKHA NAIR.** Extracellular Vesicles Derived from Preosteoblasts Influence Embryonic Stem Cell Différentiation. *Stem Cells and Development,* 2014, vol. 23 (14
- **VINCENZO CANTALUPPI.** Microvesicles Derived From Endothelial Progenitor Cells Enhance Neoangiogenesis of Human Pancreatic Islets. *Cell Transplantation.,* 2012, vol. 21, 1305-1320
- **HAE KYUNG.** Mesenchymal stem cells deliver exogenous miRNAs to neural cells and induce their différentiation and glutamate transporter expression - Delivery of miRNAs by MSCs to neural cells. *Stem Cells Dev.,* 2014, vol. 23, 2851-61
- **HONGQI XIN.** Systemic administration of exosomes released from mesenchymal stromal cells promote functional recovery and neurovascular plasticity after stroke in rats. *Journal of Cerebral Blood Flow & Metabolism,* 2013, vol. 2013, 1-5
- **JIAN-YING CHEN.** Therapeutic effects of mesenchymal stem cell-derived microvesicles on pulmonary arterial hypertension in rats-Acta. *Pharmacologica Sinica,* 04 Août 2014
- **LEE C.** Exosomes médiate the cytoprotective action of mesenchymal stromal cells on hypoxia-induced pulmonary hypertension. *Circulation.,* 27 Novembre 2012, vol. 126 (22), 2601-11
- Islam MN - Mitochondrial transfer from bone-marrow-derived stromal cells to pulmonary alveoli protects against acute lung injury - Mitochondria to the rescue. *Nat Med.,* 2012
- **CHANGJIN LEE.** Exosomes Médiate the Cytoprotective Action of Mesenchymal Stromal Cells on Hypoxia-Induced Pulmonary Hypertension. *Circulationaha.,* 31 Octobre 2012, vol. 112, 114173
- **ALIOTTA JM.** Stable cell fate changes in marrow cells induced by lung-derived microvesicles. *J Extracell Vesicles.,* 16 Avril 2012, 1
- **DER-CHERNG TARG.** Induced Pluripotent Stem Cell-Derived Conditioned Medium Attenuates Acute Kidney Injury by Downregulating the Oxidative Stress-Related Pathway in Ischemia-Reperfusion Rats. *Cell Transplantation,* 2016, vol. 25, 517-530
- **BRUNO S.** Microvesicles derived from mesenchymal stem cells enhance survival in a lethal model of acute kidney injury. *PloS One.,* 2012, vol. 7 (3), e33115
- **CRISTINA GRANGE.** Biodistribution of mesenchymal stem cell-derived extracellular vesicles in a model of acute kidney injury monitored by optical imaging. *International Journal of Molecular Medicine,* 2014, vol. 33, 1055-1063
- **GRANGE C.** Biodistribution of mesenchymal stem cell-derived extracellular vesicles in a model of acute kidney injury monitored by optical imaging. *Int J Mol Med.,* Mai 2014, vol. 33 (5), 1055-63
- **CANTALUPPI V.** Microvesicles derived from endothelial progenitor cells protect the kidney from ischemia-reperfusion injury by microRNA-dépendent reprogramming of résident renal cells. *Kidney Int.,* Août 2012, vol. 82 (4), 412-27
- **VINCENZO CANTALUPPI.** Microvesicles derived from endothelial progenitor cells protect the kidney from ischemia-reperfusion injury by microRNA-dépendent reprogramming of résident renal cells. *Kidney International advance online publication,* 11 Avril 2012
- **JOSEPH V. BONVENTRE.** Microvesicles from Mesenchymal Stromal Cells Protect against Acute Kidney Injury. *J Am Soc Nephrol,* 2009, vol. 20, 925-932

- **STEFANIA BRUNO.** Mesenchymal Stem Cell-Derived Microvesicles Protect Against Acute Tubular Injury. *J Am Soc Nephrol,* 2009, vol. 20, 1053-1067
- **JANET E.** Cranial grafting of stem cell-derived microvesicles improves cognition and reduces neuropathology in the irradiated brain. *PNAS Early Edition, www.pnas.og/cgi/doi/10.1073/pnas.1521668113*
- **KRISHNAN et al.** Engineering a growth factor embedded nanofiber matrix niche to promote vascularization for functional cardiac régénération. *Biomaterials,* 2016, vol. 97, 176-95
- **LU Y et al.** Coaxial electrospun fibers: applications in drug delivery and tissue engineering. Wiley Interdiscip Rev Nanomed Nanobiotechnol, 05 Février 2016
- **GAETANI R et al.** Epicardial application of cardiac progenitor cells in a 3D-printed gelatin/hyaluronic acid patch préserves cardiac function after myocardial infarction. *Biomaterials,* 2015, vol. 61, 339-48
- **GRAY WD et al.** Identification of therapeutic covariant microRNA clusters in hypoxia-treated cardiac progenitor cell exosomes using systems biology. *Circ. Res.,* 16 Janvier 2015, vol. 116 (2), 255-63
- **PR. PHILIPPE MENASCHÉ et al.** Towards a clinical use of human embryonic stem cell-derived cardiac progenitors : a translational expérience. *European Society of Cardiology, European Heart Journal,* 2015, vol. 36, 743-750
- Cardiovascular progenitor-derived extracellular vesicle recapitulate the bénéficiai effect of their parent cells in threatment of chronic heart failure. **ANAÏS KERVADEC et al.** Original Pre-clinic Science, The Journal of Heart and Lung Transplantation. Elsevier, 13 Janvier 2016, vol. 35, 795-807
- **SPICER et al.** Fibrin glue as a drug delivery system. *J Control Release,* 2010, vol. 148, 49-55
- **AHMAD et al.** Fibrin matrices: « The versatile therapeutic delivery systems. *International Journal of Biomolecules,* 2015, vol. 81, 121-36
- **T. CHANDY.** CP Sharma chitosane comme biomatériau Biomat. *Art. Cells, Art. Org,* 1990, vol. 18, 1-24
- **LADAGE et al.** Stimulating myocardial régénération with periostin Peptide in large mammals improves fonction post-myocardial infarction but increases myocardial fibrosis. *PLoS One.,* 2013, vol. 8, e59656
- **MORCUENDE et al.** Neuroprotective effects of NGF, BDNF, NT-3 and GDNF on axotomized extraocular motoneurons in neonatal rats. *Neuroscience,* 2013, vol. 250, 31-48
- **SKJAK-BRAERK, G ; GRASDALEN, H. ; SMIDSROD, O.** Inhomogeneous polysaccharide ionic gels. *Carbohydr. Polym.,* 1989, vol. 10, 31-54
- **MANN et al.** One-year follow-up results from AUGMENT-HF: a multicentre randomized controlled clinical trial of the efficacy of left ventricular augmentation with Algisyl in the treatment of heart failure. *Eur J Heart Fail.,* 2016, vol. 18, 314-25
- **HE. et al.** Optimizationof SDS exposure on préservation of ECM characteristics in whole organ decellularization of rat kidneys. *J Biomed Mater Res B Appl Biomater.,* 08 Avril 2016
- **MOHITI-ASLI et al.** Ibuprofen loaded PLA nanofibrous scaffolds increase prolifération of human skin cells in vitro and promote healing of full thickness incision wounds in vivo. *J Biomed Mater Res B Appl Biomater.,* 28 Octobre 2015
- **TYLER et al.** Polylactic acid (PLA) controlled delivery carriers for biomédical applications. *Adv Drug Deliv Rev.,* 15 Juillet 2016, S0169-409X
- **JAMES et al.** Poly(lactic acid) for delivery of bioactive macromolecules. *Adv Drug Deliv Rev,* 25 Juin 2016, 0169-409
- **LIU et al.** *HB-EGF embedded in PGA/PLLA scaffolds via subcritical CO2 augments the production of tissue engineered intestine. Biomaterials,* 2016, vol. 103, 150-9
- **ARIAS JA et al.** Synthesis, degradability, and drug releasing properties of methyl esters of fungal poly(beta,L-malic acid). *Macromol Biosci.,* 2008, vol. 8, 540-50
- **SINGH S et al.** The enhancement of VEGF-mediated angiogenesis by polycaprolactone scaffolds with surface cross-linked heparin. *Biomaterials,* 2011, vol. 32, 2059-2069
- Mesenchymal Stem Cells Secrete Immunologically Active Exosomes. **BIN ZHANG.** Stem Cells and Development. Mary Ann Liebert, Inc, 2014, vol. 23
- **CELINE AKYUREKLI.** A Systematic Review of Preclinical Studies on the Therapeutic Potential of Mesenchymal Stromal Cell-Derived Microvesicles. *Stem Cell Rev and Rep,* 05 Août 2014
- HucMSC-exosome Mediated - Wnt4 Signaling is Required for Cutaneous Wound Healing. **BIN ZHANG.** Stem Cells. AlphaMed Press, 2014, vol. 00, 00-00
- Extracellular Vesicles Derived from Preosteoblasts Influence Embryonic Stem Cell Différentiation. **REKHA NAIR.** Stem Cells and Development. Mary Ann Liebert, Inc, 2014, vol. 23
- **HAE KYUNG.** Mesenchymal stem cells deliver exogenous miRNAs to neural cells and induce their différentiation and glutamate transporter expression. *Delivery of miRNAs by MSCs to neural cells. Stem Cells Dev.,* 2014, vol. 23, 2851-61
- **JIAN-YING CHEN.** Therapeutic effects of mesenchymal stem cell-derived microvesicles on pulmonary arterial hypertension in rats. *Acta Pharmacologica Sinica,* 04 Août 2014

- **LEE C.** Exosomes médiate the cytoprotective action of mesenchymal stromal cells on hypoxia-induced pulmonary hypertension. *Circulation,* 27 Novembre 2012, vol. 126 (22), 2601-11
- **ISLAM MN et al.** Mitochondrial transfer from bone-marrow-derived stromal cells to pulmonary alveoli protects against acute lung injury - Mitochondria to the rescue. *Nat Med.,* 2012
- **CHANGJIN LEE.** Exosomes Médiate the Cytoprotective Action of Mesenchymal Stromal Cells on Hypoxia-Induced Pulmonary Hypertension. *Circulationaha,* 31 Octobre 2012, vol. 112, 114173
- **ALIOTTA JM.** Stable cell fate changes in marrow cells induced by lung-derived microvesicles. *J Extracell Vesicles.,* 16 Avril 2012, vol. 1
- **CANTALUPPI V.** Microvesicles derived from endothelial progenitor cells protect the kidney from ischemia-reperfusion injury by microRNA-dependent reprogramming of résident renal cells. *Kidney Int.,* Août 2012, vol. 82 (4), 412-27
- **JANET E.** Cranial grafting of stem cell-derived microvesicles improves cognition and reduces neuropathology in the irradiated brain. *PNAS Early Edition, www.pnas.og/cgi/doi/10.1073/pnas.1521668113*
- **MASAKO NAKANO.** Bone marrow-derived mesenchymal stem cells improve diabetes-induced cognitive impairment by exosome transfer into damaged neurons and astrocytes. *Scientific reports,* vol. 6, 24805
- **GRAY WD et al.** Identification of therapeutic covariant microRNA clusters in hypoxia-treated cardiac progenitor cell exosomes using systems biology. *Circ. Res.,* 16 Janvier 2015, vol. 116 (2), 255-63